# EUROPEAN PATENT SPECIFICATION

(11) **EP 1 181 320 B1**
(45) Date of publication and mention of the grant of the patent: **09.03.2011**
(21) Application number: 00930184.7
(22) Date of filing: 27.04.2000
(51) Int. Cl.: C07K 19/00, C12N 15/62, A61K 39/00, A61K 39/395, C12N 15/06, C12N 15/07, C12N 15/79, C07K 14/445, C07K 16/00

(54) **SOMATIC TRANSGENE IMMUNIZATION AND RELATED METHODS**
SOMATISCHE TRANSGENE IMMUNISIERUNG UND DARAUF BEZOGENE VERFAHREN
IMMUNISATION TRANSGENIQUE SOMATIQUE ET PROCEDES APPARENTES

(30) Priority: 27.04.1999 US 300959
(43) Date of publication of application: 27.02.2002
(73) Proprietor: Nevagen LLC, La Jolla CA 92037 (US)
(72) Inventor: ZANETTI, Maurizio, La Jolla, CA 92037 (US)
(74) Representative: UEXKÜLL & STOLBERG
(86) International application number: PCT/US2000/011372
(87) International publication number: WO 2000/064488

(56) References cited:
- XIONG SIDONG ET AL: "Engineering vaccines with heterologous B and T cell epitopes using immunoglobulin genes." NATURE BIOTECHNOLOGY, vol. 15, no. 9, 1997, pages 882-886, XP000918882 ISSN: 1087-0156
- GERLONI M. ET AL.: "Activation of CD4 T cells by somatic transgenesis induces generalized immunity of uncommitted T cells and immunologic memory" J. IMMUNOL., vol. 162, no. 7, 1 April 1999 (1999-04-01), pages 3782-3789, XP000918884
- GERLONI MARA ET AL: "Immunological memory after somatic transgene immunization is positively affected by priming with GM-CSF and does not require bone marrow-derived dendritic cells." EUROPEAN JOURNAL OF IMMUNOLOGY, vol. 28, no. 6, June 1998 (1998-06), pages 1832-1838, XP000918726 ISSN: 0014-2980
- NAKANO HIDEKI ET AL: "Genetic defect in T lymphocyte-specific homing into peripheral lymph nodes." EUROPEAN JOURNAL OF IMMUNOLOGY, vol. 27, no. 1, 1997, pages 215-221, XP000941216 ISSN: 0014-2980
- GERLONI MARA ET AL: "Durable immunity and immunologic memory to a parasite antigen induced by somatic transgene immunization." VACCINE, vol. 16, no. 2-3, January 1998 (1998-01), pages 293-297, XP004098638 ISSN: 0264-410X

## Description

### BACKGROUND OF THE INVENTION

Previous studies have shown that plasmid DNA introduced into an adult immunocompetent host could induce an antibody response (Tang et al., Nature 356:152-154 (1992)). It was soon demonstrated using the influenza virus that both humoral and cell-mediated could be induced, and these were sufficient for protection *in vivo* (Ulmer et al., Science 259:1745-1749 (1993); Fynan et al., Proc. Natl. Acad. Sci. USA 90:11478-11482 (1993)). DNA vaccines, also called genetic vaccines, have been applied to immunize against cancer (Conry et al., Cancer Res. 54:1164-1168 (1994); bacteria (Tascon et al., Nat. Med. 2:888-892 (1996); Huygen et al., Nat. Med. 2:893-898 (1996)); virus (Ulmer et al., *supra,* 1993; Fynan et al., *supra,* 1993; Raz et al., Proc. Natl. Acad. Sci. USA 91:9519-9523 (1994); Davis et al., Vaccine 12:1503-1509 (1994); Wang et al., Proc. Natl. Acad. Sci. USA 90:4156-4160 (1993); and parasites (Sedegah et al., Proc. Natl. Acad. Sci. USA 91:9866-9870 (1994)).

Genetic vaccines introduce into a host the *"blue-print"* for vaccine molecules in a way that mimics viral infections without the infectious threat. Inoculation of functional genes into somatic cells of adult immunocompetent animals is a simple way to mimic natural infection and initiate adaptive immunity (Ulmer et al., Curr. Opin. Immunol. 8:531-536 (1996)).

Plasmid DNA containing antigen-coding sequences and regulatory elements for their expression can be introduced in tissues by parenteral injection (Wang et al., *supra,* 1993) or by particle bombardment (Tang et al., *supra,* 1992). Typically, injections of plasmid DNA via the intramuscular or intradermal route yields both antibody and cellular responses with long-lasting immunity preferably induced by multiple DNA inoculations (Sedegah et al., *supra,* 1994; Xiang et al., Virology, 199:132-140, (1994)). The transgene product is, however, rarely found in the circulation (Davis et al., Human Gene Therapy, 4:151-159, (1993)), and little is known about where and how antigen presentation occurs.

Xiong et al., 1997, Nature Biotechnology 15:882-886, relates to *in vivo* DNA vaccination with a hematopoietic cell specific expression construct encoding epitopes, wherein the construct is directly injected into the spleen of mice.

Immunization via DNA inoculation relies on *in vivo* transfection, production and, when demonstrated, secretion of the transgene product, and antigen presentation by specialized cells. However, in most studies, neither the *in vivo* transfected cells nor the antigen presenting cells involved in this process have been identified. Expression of foreign DNA under the control of viral promoters (Tang et al., *supra,* 1992; Ulmer et al., *supra,* 1993; Davis et al., *supra,* 1993; Raz et al., Proc. Natl. Acad. Sci., USA, 91:9519-9523 (1994); Wang et al., *supra,* 1993; Huygen et al., *supra,* 1996; Tascon et al., *supra,* 1996; Sedegah et al., *supra,* 1994; Doolan et al., J. Exp. Med., 183:1739-1746 (1996)) limits tissue specificity.

Nakuno et al., 1997, Eur. J. Immunol.27:215-221, describes a genetic defect in a particular mouse, DDD/1, that has a paucity of lymph node cells which is due to a defect in homing to peripheral lymph nodes found to be associated with a single autosomal recessive gene designated *plt*. B cell homing to the spleen or mucosa-associated lymphoid tissue, such as Peyer's Patches, is not affected.

Although genetic vaccines have been used successfully, there remains a need to develop more effective methods to exploit their immunogenic potential. The present invention satisfies this need and provides related advantages as well.

### SUMMARY OF THE INVENTION

The invention provides use of B lymphocytes obtained from an individual or from an immunologically compatible individual to which nucleic acid molecules have been administered ex *vivo,* wherein the nucleic acid molecules comprise a hematopoietic cell-specific expression element operationally linked to a nucleic acid sequence encoding one or more heterologous epitopes, for the preparation of a pharmaceutical composition for administration to the individual for stimulating an immune response.

The invention also provides a nucleic acid molecule comprising a hematopoietic cell-specific expression element which functions in a B cell operationally linked to a nucleic acid sequence encoding a heterologous polypeptide, wherein said heterologous polypeptide comprises two or more T cell epitopes, wherein said T cell epitopes are selected from the group consisting of a CD4 and a CD8 epitope, two CD4 epitopes, and two CD8 epitopes.

### BRIEF DESCRIPTION OF THE DRAWINGS

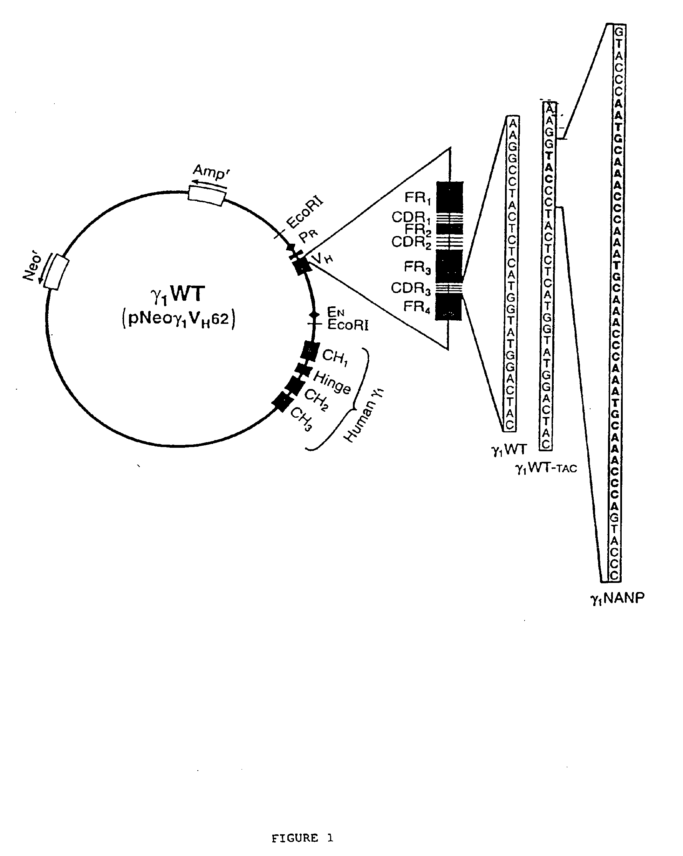

Figure 1 shows a schematic representation of plasmid DNA y1WT and its y1WT-TAC and y1NANP variants. The γ1WT H chain construct is the product of the fusion between a human γ1 constant (C) region gene present in the plasmid vector pNeoy1 with the murine V_{H}62 gene (2.3 kb) (Sollazzo et al., Eur. J. Immunol., 19:453-457 (1989)). The V_{H} region gene is productively rearranged and the C region gene is in genomic configuration. Variants y1WT-TAC and γ1NANP contain the nucleotide insertions shown in bold characters in CDR3. Each plasmid DNA carries the regulatory elements, promoter (Pr) and enhancer (En) needed for tissue-specific expression. In plasmid DNA γ1NANP the human γ1 C region gene is joined to a productively rearranged murine variable (V) region gene modified in the third complementarity determining region (CDR3) by introduction of the nucleotide sequence coding for three Asn-Ala-Asn-Pro repeats. In these plasmids, the promoter and enhancer elements are those constitutively existing in Ig H chain genes. Neo^{r}=neomycin resistance gene; Amp^{r}=ampicillin resistance gene; PR=promoter; EN=enhancer; C_{H}=heavy chain C region; V_{H}=heavy chain variable region; FR=framework region; CDR=complementarity determining region.

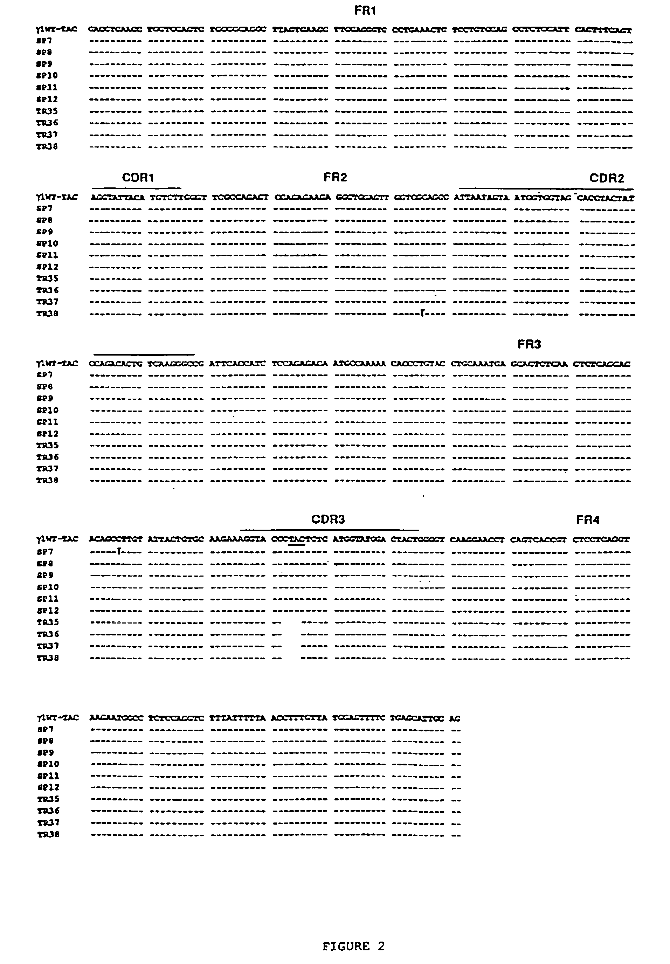

Figure 2 shows the nucleotide sequence of genomic DNA clones corresponding to the productively rearranged VDJ region of γ1WT-TAC DNA. A 520 bp fragment was amplified from (1) genomic DNA extracted from a spleen inoculated 17 days earlier with plasmid DNA γ1WT-TAC, and (2) J558L cells constitutively harboring plasmid DNA γ1WT (Sollazzo et al., *supra,* 1989). The amplified products were cloned and sequenced using two different primers from opposite directions. The top nucleotide sequence refers to γ1WT-TAC and serves as a reference. SP7-SP12 identify six clones originated from splenic genomic DNA. TR35-TR38 identify four genomic DNA clones derived from transfectoma cells. The CDR and framework regions (FR) are indicated. This study indicates that after injection *in vivo* the transgene does not undergo somatic mutation.

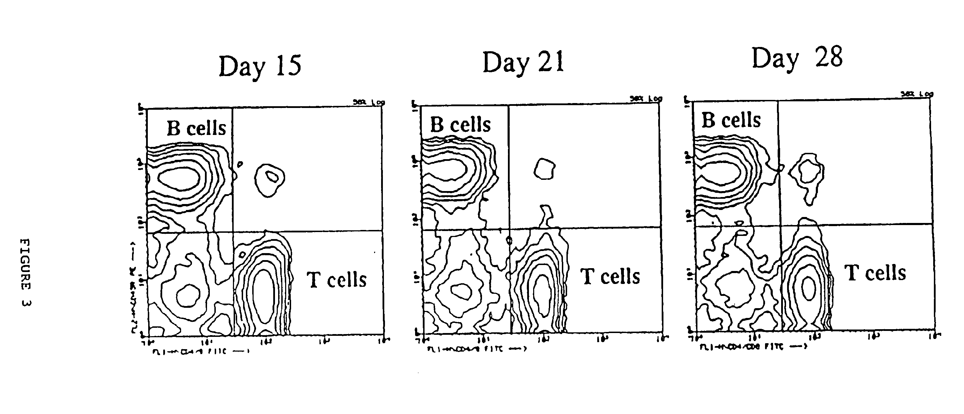

Figure 3 shows isolation of splenic B and T lymphocytes and detection of the transgene H-chain in the purified lymphocyte populations. B and T lymphocytes from the spleen of DNA-inoculated mice were sorted and purified on a fluorescence-activated cell sorter at the times indicated.

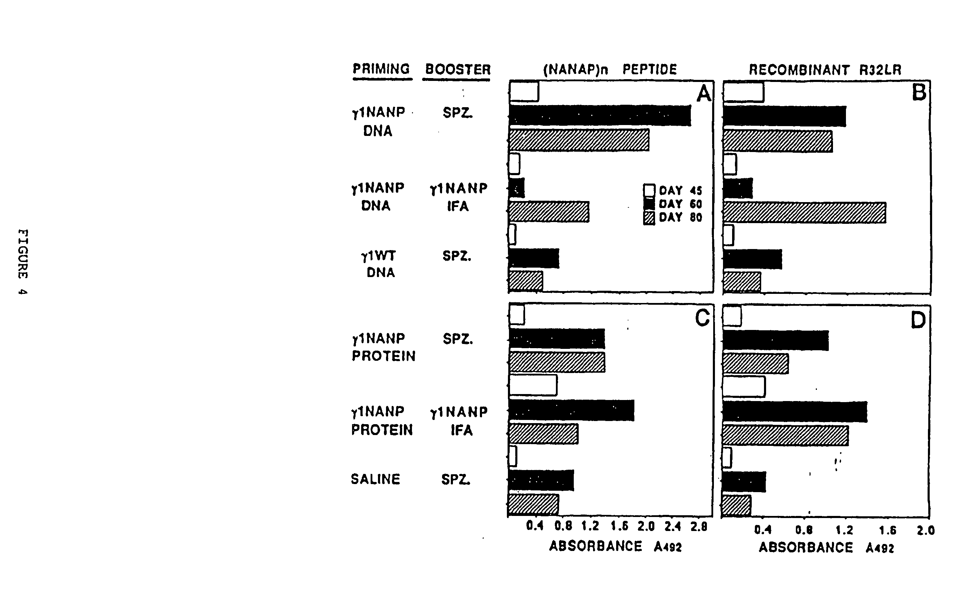

Figure 4 shows the anamnestic response elicited with plasmid _{Y}1NANP DNA following challenge with *P. falciparum* sporozoites. Mice were primed with plasmid DNA _{Y}1NANP or antigenized antibody _{Y}1NANP or antigenized antibody γ1NANP in CFA as indicated. Control groups were inoculated with plasmid _{Y}1WT DNA or saline. On day 45 mice were given a booster immunization with either *P. falciparum* sporozoites or antigenized antibody γ1NANP (50 µg) in IFA subcutaneously as indicated. *P. falciparum* sporozoites were inoculated (10⁹) in incomplete DMEM intraperitoneally. Blood samples were collected on day 45 (before the booster injection) and subsequently 15 and 35 days after booster. Antibodies reactive with the synthetic peptide (NANP)n (panels A and C) and antibodies reactive with the recombinant protein R32LR (panels B and D) were detected by ELISA. Values represent the absorbance (A₄₉₂) of pooled sera (four mice/group) tested at 1:1600 dilution.

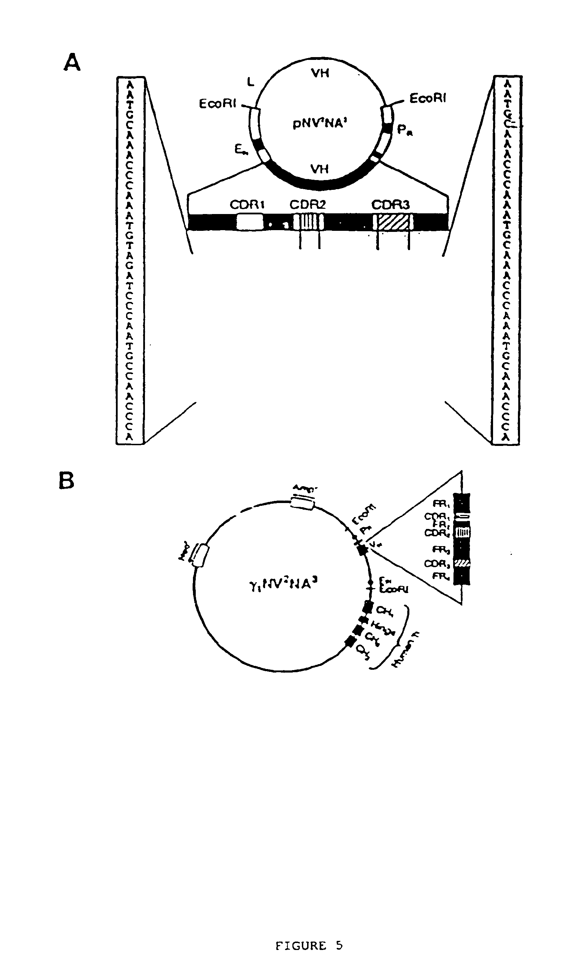

Figure 5 shows engineering and expression of an immunoglobulin H chain gene with two heterologous epitopes. Panel A shows a schematic representation of the mutagenesis vectors, introduction of the (NANP)3 and NANPNVDPNANP coding sequences and partial, nucleotide sequence of CDR2 and CDR3 after insertion. The synthetic oligonucleotides and the mutagenesis steps for the creation of pVH-TAC/CCA are detailed in the Experimental Protocol. Two pairs of complementary synthetic oligonucleotides coding for (NANP)3 and NANPNVDPNANP, were cloned in the Asp718 site in CDR3 and in the NcoI site in CDR2 of pVH-TAC/CCA. The insertions were verified by dideoxy- chain-termination sequencing. Panel B shows a schematic representation of plasmid DNA γ1NV²NA³ carrying the coding sequences for the two heterologous epitopes in CDR3 and CDR2, respectively. The human γ1 constant (C) region gene is in genomic configuration. CH1, CH2, and CH3 refers to the corresponding domains in the C region of the y1 gene. Promoter (Pr) and enhancer (En) elements for tissue-specific expression and the neomycin (Neo^{r}) and ampicillin (Amp^{r}) resistance genes are indicated. Panel C shows a schematic representation of antigenized H chain gene product paired with a light chain. The engineered epitopes in CDR3 and CDR2 are as indicated (not to scale).

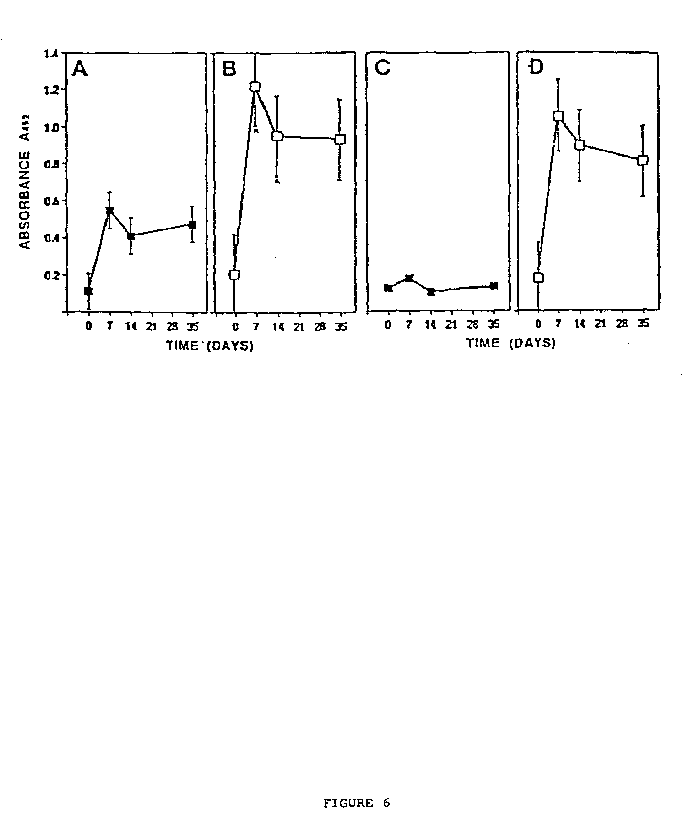

Figure 6 shows *in vivo* immunogenicity of CDR3 and CDR2 epitopes. Mice were immunized with plasmid DNA _{Y}1NANP (black squares) or γ1NV²NA³ (open squares). Their sera were tested by ELISA on synthetic peptide (NANP)n (panels A and B) or NANPNVDPNANP (panels C and D). Values refer to absorbance (492 nm) of sera tested at 1:1600 dilution and are expressed as the mean (± standard error). Each group consisted of four mice. (*) indicates statistical significance between the values shown in panel B versus panel A. Significance was p<0.01 on day 7, and p<0.05 on day 14. Time refers to days after DNA inoculation.

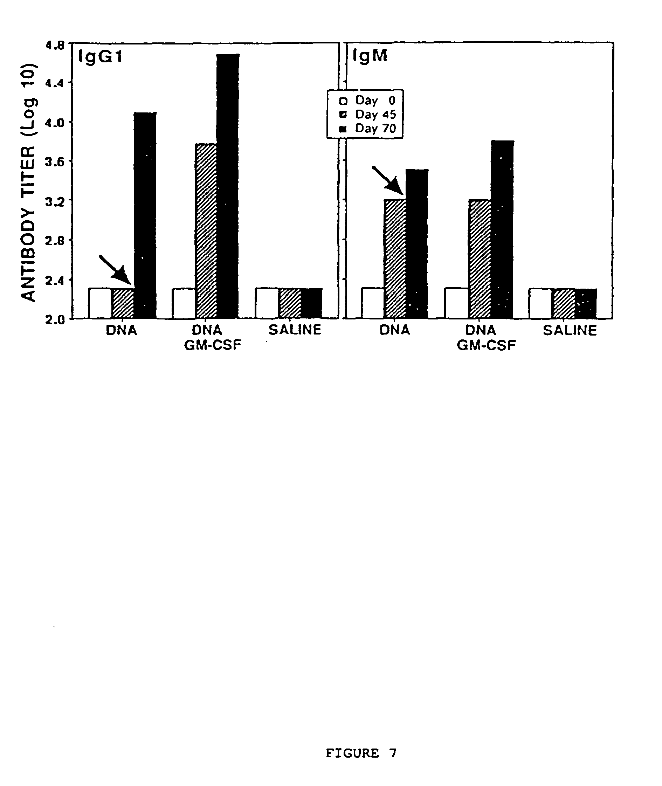

Figure 7 shows GM-CSF heightens the anamnestic anti-NANP antibody response following booster immunization with *P. falciparum* sporozoites. Columns refer to antibody titers (Log 10) were measured on (NANP)n peptide. Experimental groups are identified at the bottom. The arrow indicates the time (day 45) when the booster immunization was given. Values refer to binding of a pool of sera collected at the same time. Each group consisted of four mice.

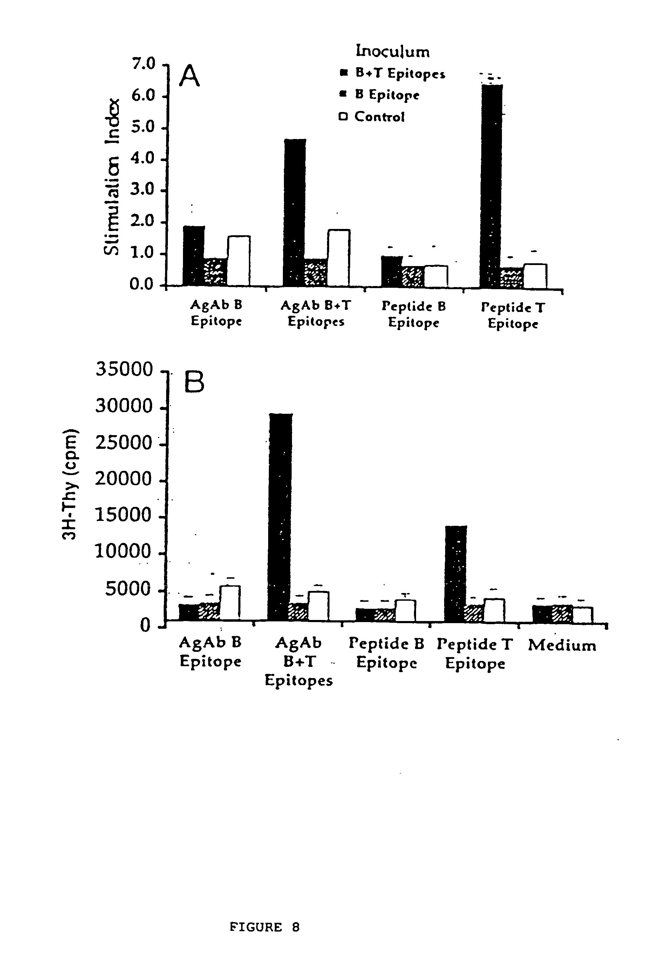

Figure 8 shows antigen-specific activation of T lymphocytes by STI. Panel A shows the proliferative response of spleen cells from C57B1/6 mice inoculated with plasmid DNA g1NANP coding for the B cell epitope (4 mice), γ1NV²NA³ coding for the B and T cell epitopes (4 mice), or control plasmid pSV2neo (2 mice), and harvested on day 7. Cells were cultured in the presence of the antigens indicated along the abscissa. Results refer to stimulation index expressed as the mean ± S.D.. Results correspond to two independent experiments. AgAb = antigenized antibody. Tests were run in triplicate. Panel B shows IL-2 production in spleen cell cultures from the same C57B1/6 mice shown in panel A. Results are expressed as counts per minute (cpm) of the proliferative response of indicator NK.3 cells and are expressed as the mean ± S.D.

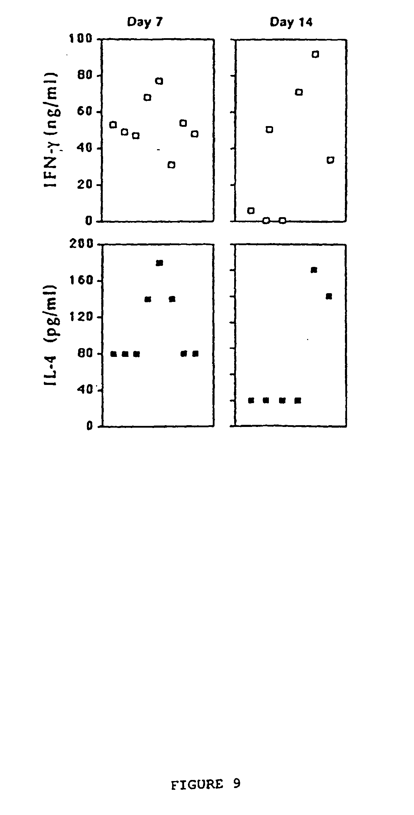

Figure 9 shows levels of IFN-_{Y} and IL-4 during the primary response. Spleen cells harvested 7 and 14 days after immunization were incubated with synthetic peptide corresponding to the Th cell determinant (50 γg/ml) for 40 hours. Supernatants from triplicate cultures were harvested and tested in capture ELISA specific for IFN-_{Y} or IL-4.

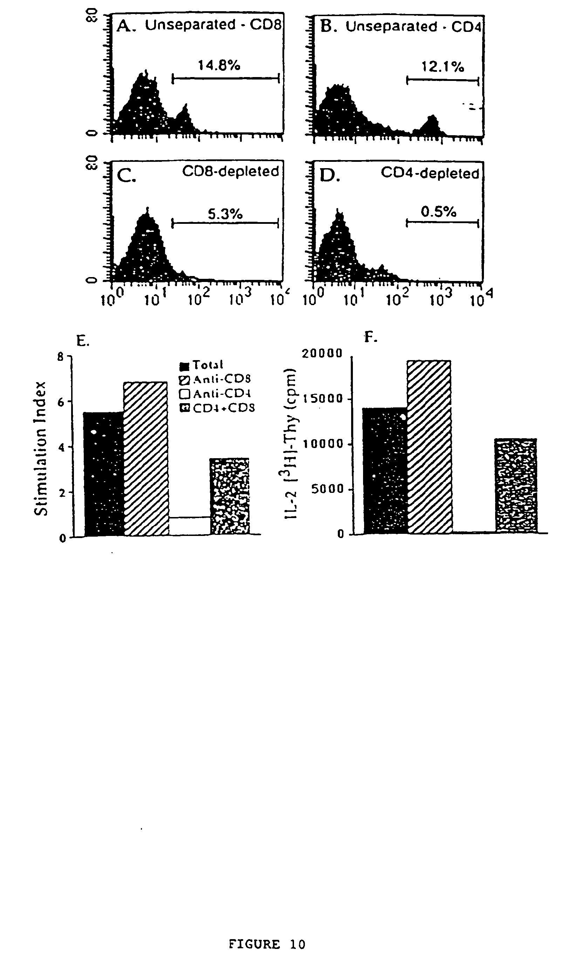

Figure 10 shows activated cells are CD4⁺T cells. Seven days after DNA inoculation, spleen cell populations were prepared and depleted of CD8⁺ (Panel C) or CD4⁺ (Panel D) cells by antibody plus complement. Unseparated CD8⁺ cells (Panel A) and unseparated CD4⁺ cells (Panel B) are shown as reference. The proliferative response (Panel E) and IL-2 production (Panel F) of unfractionated (total), separated CD4 and CD8, and reconstituted (CD4+CD8) T cell populations are shown. Stimulation indexes and IL-2 production were determined.

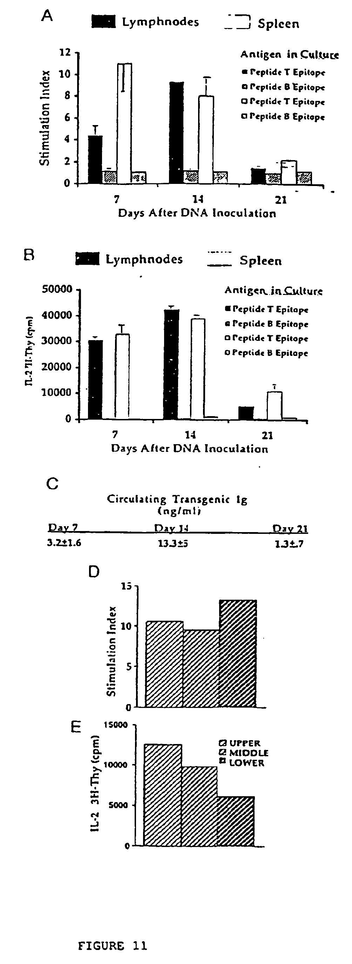

Figure 11 shows T cell immunity induced by intraspleen DNA inoculation spreads to lymph nodes. Cell proliferation (Panel A) and IL-2 production (Panel B) in a pool of inguinal, mesenteric and cervical lymph node, and spleen cells harvested 7, 14 or 21 days after γy1NV²NA³ DNA inoculation. Lymph nodes were isolated from four mice/experiment. Serum transgenic Ig (ng/ml) in the serum is expressed as the mean ± SD of six different mice at each time point (Panel C). Cell proliferation (Panel D) and IL-2 production (Panel E) of lymph nodes collected from (1) axillary, brachial, deep and superficial cervical (upper); (2) mesenteric, renal and epigastric (middle): and (3) popliteal, caudal, sciatic and lumbar (lower), lymph nodes 14 days after DNA inoculation. Lymph nodes were isolated from six mice.

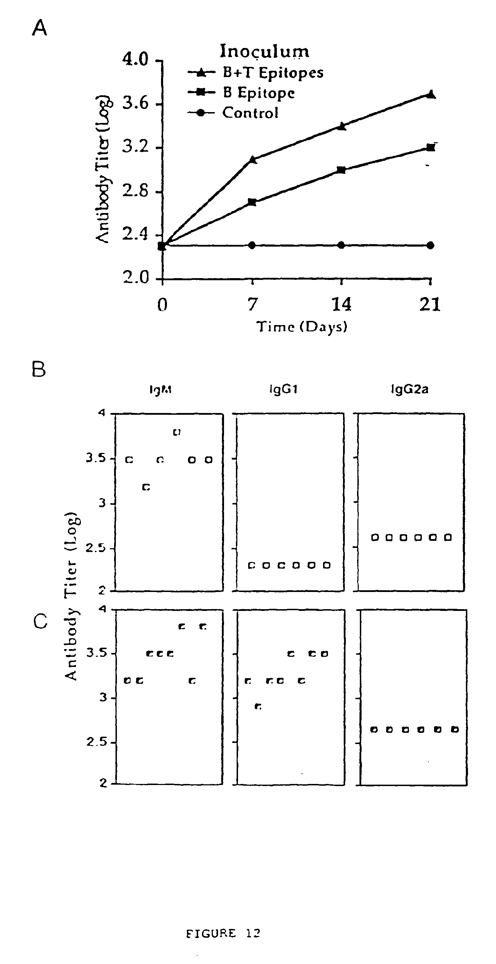

Figure 12 shows the effect of linked recognition of Th and B cell epitopes on the antibody response. Titer (Log) of B-cell epitope reactive antibodies in mice inoculated with plasmid DNA coding for T and B epitopes (triangle), B cell epitope (square) or control plasmid (circle) (Panel A). The titer (Log) of IgG1, IgM and IgG2a antibodies determined in ELISA in the sera of mice inoculated with plasmid DNA coding for the B-cell epitope only (Panel B) or with plasmid DNA coding for the B- and T cell epitopes (Panel C). Every symbol refer to a single mouse. All mice were tested on day 14. Tests were done in duplicate.

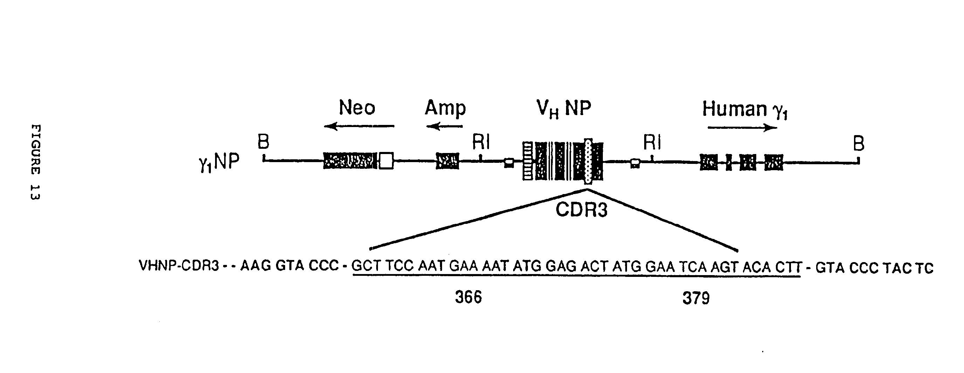

Figure 13 shows a schematic representation of plasmid DNA γ1NP. This H-chain coding plasmid is the product of the fusion of a human γ1C region with a murine VH engineered to express the 13 amino acid residues from the sequence of the influenza virus nucleoprotein (NP) antigen (366-379) in the third complementarity-determining region (CDR3). This NP peptide is presented in association with the Db allele in H-2b mice. The coding strand of the CDR3 region is shown in bold, with the NP-coding sequence underlined. The amino acid sequence of the influenza peptide 366ASNENMETMESSTL379 is shown in bold. B, BamHI; RI, EcoRI; Neo, neomycin (G418) resistance; Amp, ampicillin resistance. The H-chain gene was mutagenized to introduce a single KpnI/Asp718 site and complementary oligonucleotides 5' GTA CCC GCT TCC AAT GAA AAT ATG GAG ACT ATG GAA TCA AGT ACA CTT 3', 5' GTA CAA GTG TAC TTG ATT CCA TAG TCT CCA TAT TTT CAT TGG AAG CGG 3' coding for residues 366-379 of the influenza virus NP antigen (ASNENMETMESSTL) were introduced between 94V and 95P of the mutagenized VH region. The engineered VHNP coded by the 2.3 kb EcoRI fragments was cloned upstream from a human γ1 constant (C) region gene contained in the 12.8 kb vector pNγ1

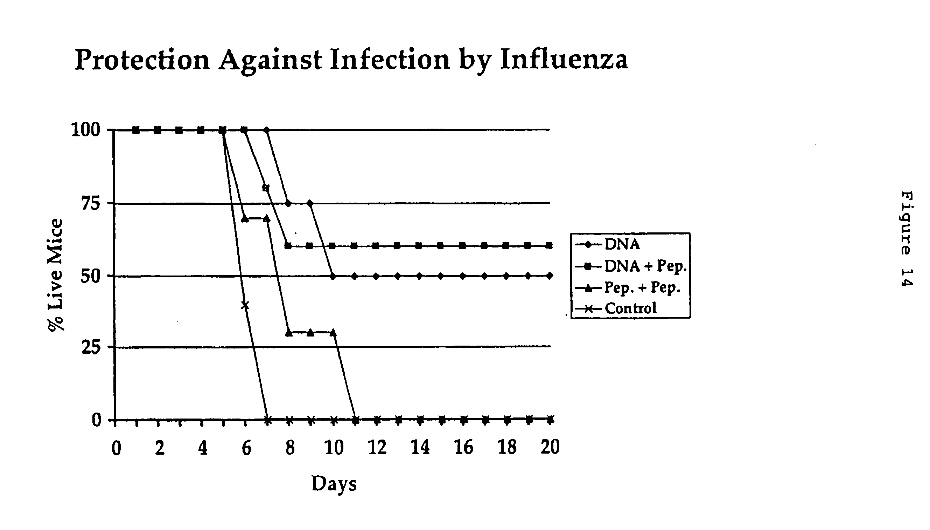

Figure 14 shows survival curves in mice vaccinated with plasmid DNA _{Y}1NP (DNA) via intraspleen inoculation and challenged with ×10LD₅₀ influenza virus. Other groups were primed with plasmid DNA γ1NP followed by a booster with synthetic peptide the influenza virus NP antigen ASNENMETMESSTL in immunologic adjuvant (DNA + peptide), or NP synthetic peptide ASNENMETMESSTL in immunologic adjuvant followed by a booster with the same synthetic peptide (peptide + peptide). Challenge with the virus was given three months after priming.

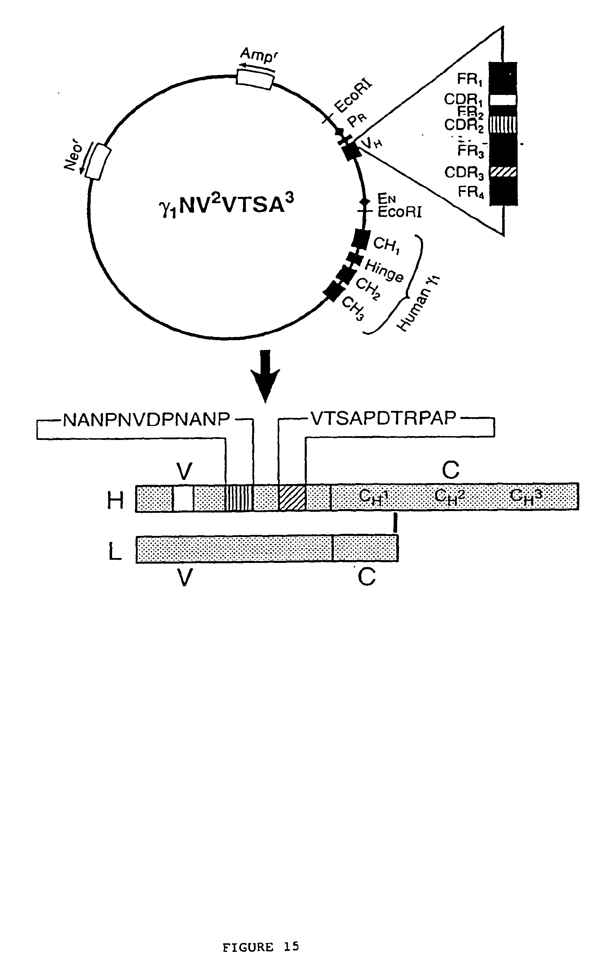

Figure 15 exemplifies the engineering of an immunoglobulin H chain gene with two heterologous Th cell epitopes. The H chain gene is coded by plasmid vector _{Y}1NV2VTSA3. The VH region is the 2.3 kb Eco RI genomic fragment containing the VDJ rearrangement of a murine V region gene (see Figure 1 for detail). The human γ1 constant (C) region gene is in genomic configuration. CH1, CH2, and CH3 refers to the corresponding domains in the C region of the γ1 gene. Promoter (Pr) and enhancer (En) elements for tissue-specific expression and the neomycin (Neo^{r}) and ampicillin (Amp^{r}) resistance genes are indicated. The VH region is modified by mutagenesis to code for two heterologous determinants as indicated in the right panel. The arrow points the structure of the translated protein composed of the transgenic H chain and a light (L) chain provided by the host cell. The amino acid sequences in the CDR2 and CDR3, are indicated and correspond to the Th cell determinant NANPNVDPNANP from the outer coat of the malaria parasite *P. falciparum* (in CDR2) and the VTSAPDTRPAP epitope from the tandem repeat of the tumor antigen MUC-1 (in CDR3). CDR= complementarity determining region. H = heavy (chain); C = constant region. Not to scale.

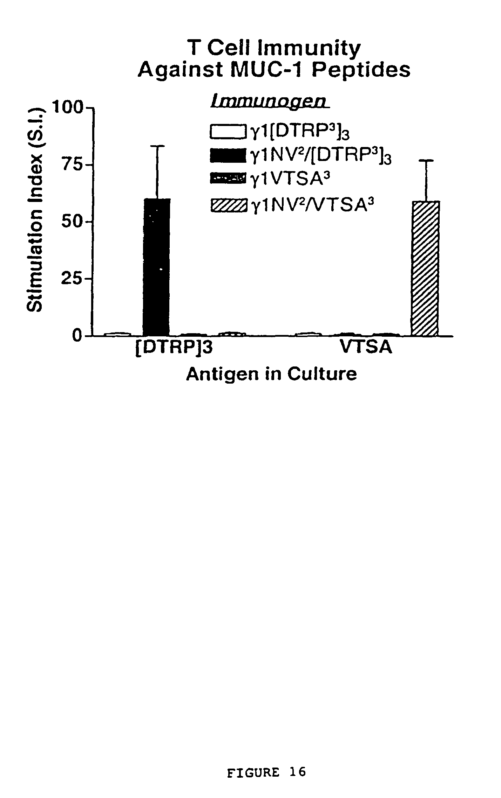

Figure 16 shows the effect of linked recognition of a dominant Th epitope and a cryptic/subdominant Th epitope on the proliferative response to the cryptic/subdominant epitope. Th/Th associative recognition is necessary to render immunogenic T cell determinant from the MUC-1 antigen. Mice were inoculated with plasmid DNA as indicated. Spleen cells were harvested on day 15 and re-stimulated *in vitro* for 4 days in the presence of 50 µg/ml of synthetic peptide (DTRP)3 and VTSAPDTRPAP (denoted as VTSA). Both sequences are contained in the PDTRPAPGSTAP tandem repeat of the tumor antigen MUC-1. Superscript numbers indicate the CDR in which the heterologous antigen sequence has been inserted. Subscript numbers indicate the number of times the sequence in brackets is repeated in the context of a particular CDR. The results shown are cumulative of three independent experiments. Each group is constituted of 8-10 mice. Results are expressed as stimulation index. Bars indicate means of stimulation indexes ± SEM.

### DETAILED DESCRIPTION OF THE INVENTION

The present invention provides a rational and effective approach to immunization and is predicated on the induction of antibody (B cell immunity) and cellular (T cell immunity) responses following inoculation of a polypeptide encoded by a nucleic acid molecule, for example, an immunoglobulin H chain gene, targeted to matopoie B lymphocytes. Immunization can be obtained by transfecting B lymphocytes, *ex vivo*, for example by the intravenous injection of lymphocytes transfected in vitro. The uses of the invention can be used to initiate immunity, establish immunologic memory and program the immune response in a reproducible way from a single inoculation of a nucleic acid molecule such as plasmid DNA.

The uses of the invention are based on an effective method for delivering a nucleic acid molecule, which can serve as a vaccine, to B cells, *ex vivo*. Transfected B cells produce amounts of immunogenic molecules and program the immune system for the immune response. The method for delivering a nucleic acid molecule such as a DNA vaccine to B cells is termed somatic transgene immunization (STI).

Specifically, STI reaches two objectives: exploit B lymphocytes as powerful minifactories of antigenic material and use them as antigen-presenting cells (APC). STI induces immunity using B cells for the protracted manufacturing of immunogenic molecules (a B cell can produce 10³ molecules of antibody/second (Langman and Cohn, Mol. Immunol. 24:675-697 (1987)). Therefore, efficient utilization of the foreign DNA and antigen presentation by the very cells harboring the transgene is addressed in one operational event. Thus, the targeting of nucleic acid molecules encoding a heterologous epitope to a lymphoid tissue exploits the natural high level expression of immunoglobulins in B lymphocytes.

The uses of the invention are effective at stimulating an immune response because the nucleic acid molecule is targeted to hematopoietic cells, namely B lymphocytes. The effectiveness of the methods result from the self-renewing property of antigenized antibody genes harbored in B lymphocytes and the constitutive ability of activated B lymphocytes to synthesize many copies of transgene products.

In one embodiment, the variable region of antibodies can be re-engineered to code for discrete sequences of heterologous antigens to impart to the molecule new antigenic and immunogenic properties, called antibody antigenization. This approach allows modification of the complementarity determining regions (CDR) of the variable domain of an immunoglobulin so that, after antigenization, antibodies become structural mimics of antigens in a way that leads to induction of B-cell and T-cell immunity. Consequently, inoculation of antigenized H chain genes and synthesis of transgenic Ig by the host during STI is a way to provide the organism with heterologous B-cell and T-cell epitopes. Methods of generating antigenized immunoglobulins is described, for example, in U.S. patents 5,583,202, issued December 10, 1996, and 5,658,762, issued August 19, 1997.

The present invention provides the combined use of STI and antigenized antibody genes as a method to induce antigen-specific immunity, antibody and T cell mediated. In addition to antigenized antibodies, the methods of the invention for stimulating an immune response can use a nucelic acid molecule expressing one or more heterologous polypeptides. The heterologus polypeptide is operationally linked to an expression element allowing expression of the polypeptide in B lymphocytes. Similar to an antigenized antibody, the methods exploit the polypeptide expression capabilities of hematopoietic cells targeted upon administration of a nucleic acid molecule to a lymphoid cell. The heterologous polypeptide can encode one or more epitopes capable of eliciting an immune response.

The uses of the invention are useful, for example, for stimulating an immune response against infectious agents, microbial pathogens, tumor antigens and pathological processes. The present invention can be used to stimulate an immune response against infectious agents including, viruses, for example, immunodeficiency virus 1 and 2, hepatitis viruses, papilloma virus, influenza virus, Epstein-Barr virus, cytomegalovirus, Japanese encephalitis virus, Dengue virus, and other retroviruses/lentiviruses; protozoa, for example, parasites causing malaria, leishmaniasis, trypanosomiasis, filariasis, toxoplasmosis, hookworm, tapeworm; yeast, for example, Candida *albicans,* bacteria, in particular pathogenic bacteria such as *Mycobacterium tuberculosis, Mycobacterium leprae,* and bacteria that cause colera, *Mycoplasma*/*Ureaplasma,* and spirochetes such as treponema pallidum, borrelia, leptospira; toxins, for example, botulinum, anthrax, snake toxins, insect toxins, and warfare-related chemical toxins.

The invention can also be used to stimulate an immune response to pathological or disease conditions. The pathological or disease conditions can be, for example, tumors, including those expressing antigens such as prostate specific antigen (PSA), Her-2/neu, p53, MUC-1, telomerase, carcinoembryonic antigen (CEA), melanoma associated antigens (MAGE), thyrosinase, gp100; autoimmune diseases, for example, diabetes, myasthenia gravis, multiple sclerosis, rheumatoid arthritis, Crohn's disease, uveitis; allergy, for example, dermatitis and athsma; metabolic disorders, for example, hypertension, diabetes, hypercholesterolemia; endocrine disorders, for example of the thyroid, adrenals, pituitary, ovary, testis; mental disorders, for example, bipolar disorders, schizophrenia; pain, for example, modulation of neurotransmitters and neuropeptides; blood disorders, for example, coagulation, anemias, thrombocytopenia; and dental disorders, for example, caries. The methods of the invention can also be used to control reproduction, for example, contraceptive vaccination. The invention can additionally be used for treating transplant patients, for example, solid organ by inducing transplantation, and bone marrow transplantation, anti-HLA immunity. The present invention can be used for the production of human and animal vaccines against viruses, parasites, bacteria, allergy, autoimmune disease, and tumors. The uses of the invention are useful for stimulating an immune response to treat or prevent a condition as described above.

The uses of the invention include the step of administering a nucleic acid molecule encoding one or more heterologous epitopes to B cells, either in *vitro.*

The secondary lymphoid tissue can be spleen, lymph nodes, mucosa-associated lymphoid tissue (MALT), including tonsils and Payer's patches, and the nasal-associated lymphoid tissue (NALT) such as the Waldeyer's ring, and the urogenital lymphoid tissue. A variety of methods can be used to administer a nucleic acid molecule to a lymphoid tissue. For example, a nucleic acid molecule can be directly injected into a lymphoid tissue such as a lymph node. A nucleic acid molecule can also be directly injected into the spleen of an individual, for example, using endoscopy-guided fine needle injection. Additional methods include the intravenous injection of DNA encapsulated into (immuno)-liposomes or biodegradable beads of various chemical structure for time-controlled release, for example, hyaluronic acid. Additional methods include the (intra)-nasal delivery of DNA encapsulated into (immuno)-liposomes or biodegradable beads or various chemical structure for time-controlled release such as hyaluronic acid. Additional methods include the oral delivery of DNA encapsulated into (immuno)-liposomes or biodegradable beads or various chemical structure for time-controlled release, for example, hyaluronic acid, in a suitable acid-resistant pharmaceutical vehicle, or engineered in live attenuated bacteria, for example, *Salmonella typhi.*

As used herein, the term "epitope" refers to a molecule or fragment thereof capable of stimulating an immune response. A polypeptide epitope is at least three amino acids in length for antibody responses and at least eight amino acids in length for T cell responses.

As used herein, the term "heterologous polypeptide" when used in reference to a nucleic acid molecule means that the polypeptide is encoded by a nucleic acid sequence operationally linked to an expression element, where the polypeptide is not naturally found linked to the expression element. As such, the polypeptide is heterologous to the expression element.

Similarly, the term "heterologous epitope" refers to an epitope encoded by a nucleic acid sequence operationally linked to an expression element, where the epitope is not naturally found linked to the expression element. When a heterologous epitope is contained in an immunoglobulin, the epitope is not ordinarily found in the immunoglobulin. Hence, the immunoglobulin contains a heterolgous epitope sequence. Such heterologous epitope sequences can include antigenic epitopes as well as receptor-like binding domains or binding regions that function as receptor sites, for example, the human CD4 or CCR5 binding domain for HIV, hormone receptor binding ligands, retinoid receptor binding ligands, and ligands or receptors that mediate cell adhesion.

The epitope encoded by the nucleic acid molecules of the invention is operationally linked to an expression element. As used herein, an "expression element" is a nucleic acid regulatory element capable of directing expression of a genetic element such as a nucleic acid encoding an epitope. An expression element can include, for example, promoters and/or enhancers capable of allowing expression of an operationally linked genetic element such as a genetic element encoding a polypeptide or epitope. Particularly useful promoters and enhancers are those that function in hematopoietic cells, termed "hematopoietic cell expression elements." Such hematopoietic expression elements are capable of allowing expression in a cell of hematopoietic origin, for example, a B cell or T cell. These promoters and enhancers can be specific for a hematopoietic cell, for example, a B cell or T cell. As used herein, a "hematopoietic cell-specific expression element" refers to an expression element that is specific for a hematopoietic cell or a particular hematopoietic cell such as a B cell-specific or T cell-specific promoter and/or enhancer. Exemplary B cell-specific expression elements are disclosed in the Examples. One skilled in the art knows or can readily determine a hematopoietic cell-specific expression element. The hematopoietic cell-specific expression element can be an expression element that occurs naturally in a hematopoietic cell such as a B cell or T cell.

The nucleic acid molecule used in the invention can encode an immunoglobulin molecule containing one or more heterologous epitopes. The epitopes can be inserted into a complementarity-determining region (CDR) of the immunoglobulin molecule (see, for example, Kabat et al., Proteins of Immunological Interest, U.S. Department of Health and Human Services, Bethesda MD (1987)). The epitope can be inserted within CDR1, CDR2 and/or CDR3. Furthermore, one or more epitopes can be inserted within any of the CDRs. Thus, the same epitope can be inserted multiple times within a single CDR or can be inserted multiple times within different CDRs. Different epitopes can also be inserted within the same CDR or can be inserted within different CDRs. Thus, a single CDR can have a single epitope, multiple copies of the same epitope, or two or more different epitopes in the same CDR. It is likely that as many as 6 epitopes, or possibly more, can be inserted into the three CDRs of a variable region of one Ig polypeptide chain. These methods utilize antigenized immunoglobulins which are described in U.S. patents 5,583,202 and 5,658,762.

Generally, when more than one epitope is administered to stimulate an immue response, the multiple epitopes are encoded on the same nucleic acid molecule. When encoded on the same plasmid, the multiple epitopes can be operationally linked to the same expression element and expressed as a fusion polypeptide, or the multiple epitopes can be expressed from multiple copies of the expression element. Multiple epitopes can also be expressed from different expression elements. Furthermore, the same epitope can be administered in different nucleic acid molecules such as different plasmids. Similarly, different epitopes can be administered in one nucleic acid molecule or can be administered in multiple nucleic acid molecules such as on different plasmids. Using different nucleic acid molecules encoding multiple epitopes allows the administration of many more epitopes than could be encoded on a single nucleic acid molecule.

The immunoglobulin molecules useful in the invention can contain the variable region of a heavy or light chain, or a functional fragment thereof. For example, a single CDR can be a functional fragment if the immunoglobulin, as used herein as an antigenized antibody, functions to stimulate an immune response. The immunoglobulin can also comprise two or three CDRs of a variable region as described above. Additionally, the immunoglobulin molecules useful in the invention can be a heavy chain or a light chain. The effector function of the immunoglobulin molecule can be conferred by the constant region of the immunoglobulin molecule. Therefore, the immunoglobulin molecule can include a constant region. The constant region can be derived, for example, from human, primate, mouse, rat, chicken or camel, as desired. However, it is understood that a constant region is not required for the immunoglobulin of the invention if the functional fragment of the immunoglobulin functions to stimulate an immune response.

The invention also provides a nucleic acid molecule comprising, a hematopoietic cell-specific expression element, which functions in a B cell operationally linked to a nucleic acid sequence encoding one or more heterologous polypeptides comprising two or more T cell epitopes. The heterologous polypeptide can function as one or more epitopes. Furthermore, the epitopes can be expressed as a fusion with a cytokine, When an epitope is expressed as a fusion polypeptide, for example, a fusion with a cytokine, the epitope can be fused proximal to a cytokine, or there can be intervening sequence between the epitope and the cytokine. The cytokine can be, for example, GM-CSF, IL-2, IL-4, INF-_{Y}, IL-5, IL-6, IL-10 and IL-12. The expression element of the nucleic acid molecules of the invention is a hematopoietic expression element, which functions in B cell.

Additionally disclosed is a method for stimulating an immune response, comprising administering *ex vivo* to a lymphoid cell a nucleic acid molecule comprising a hematopoietic cell-specific expression element operationally linked to a nucleic acid sequence encoding one or more heterologous epitopes. The lymphoid cell can be derived from blood or a lymphoid tissue selected from the group consisting of spleen, lymph nodes, mucosa-associated lymphoid tissue (MALT), tonsils, Payer's patches, nasal-associated lymphoid tissue (NALT), Waldeyer's ring, and urogenital lymphoid tissue.

Further disclosed is a method for stimulating an immune response, comprising administering to a lymphoid cell a nucleic acid molecule comprising a hematopoietic cell-specific expression element operationally linked to a nucleic acid sequence encoding one or more heterologous epitopes, wherein the lymphoid cell is in blood or a lymphoid tissue selected from the group consisting of lymph nodes, mucosa-associated lymphoid tissue (MALT), tonsils, Payer's patches, nasal-associated lymphoid tissue (NALT), Waldeyer's ring, and urogenital lymphoid tissue.

Also disclosed is a method for stimulating an immune response, comprising administering to a lymphoid tissue a nucleic acid molecule comprising an expression element, for example, a hematopoietic cell-specific expression element, operationally linked to a nucleic acid sequence encoding one or more heterologous epitopes. The lymphoid tissue can be selected from the group consisting of spleen, lymph nodes, mucosa-associated lymphoid tissue (MALT), tonsils, Payer's patches, nasal-associated lymphoid tissue (NALT), Waldeyer's ring, and urogenital lymphoid tissue.

The invention can be used to stimulate an immune response. The immune response elicited can be an antibody response, a CD4 T cell response or a CD8 T cell response. Two major classes of T cells, termed T helper cells and T cytotoxic cells, can be distinguished. The classification of T cells into T helper cells and T cytotoxic cells is generally based on the presence of either CD4 or CD8 protein, respectively, on the cell surface. The invention can be used to elicit an antibody response, a CD4 T cell response or a CD8 T cell response, or any combination of two or more of these responses, including all three responses. For example, the invention can be used to stimulate an antibody response and a CD4 T cell response. The invention can also be used to stimulate an antibody response and a CD8 T cell response. Additionally, the invention can be used to stimulate a CD4 T cell response and a CD8 T cell response. Furthermore, the invention can be used to stimulate an antibody response, a CD4 T cell response and a CD8 T cell response. In addition, the invention can be used to stimulate multiple CD4 T cell responses, for Example, two or more, three or more, or five or more CD4 T cell responses. Similarly, multiple CD8 T cell responses can be stimulated using the invention. Thus, depending on the type of immune response desired for a given type of antigen or condition, one skilled in the art can select the most appropriate immune response, an antibody, CD4 T cell or CD8 T cell response, to provide an optimized immune response for a given condition or potential condition.

The invention also provides a nucleic acid molecule comprising a hematopoietic cell-specific expression element which functions in a B cell operationally linked to a nucleic acid sequence encoding a heterologous polypeptide, wherein the heterologous polypeptide comprises two or more T cell epitopes, the T cell epitopes being selected from the group consisting of a CD4 and a CD8 epitope, two CD4 epitopes, and two CD8 epitopes. The heterologous polypeptide can further comprise one or more B cell epitopes.

The invention further provides a nucleic acid molecule comprising a hematopoietic cell-specific expression element which functions in a B cell operationally linked to a nucleic acid sequence encoding one or more heterologous epitopes, wherein the nucleic acid sequence encodes an immunoglobulin molecule containing the one or more epitopes and wherein the one or more epitopes is inserted within a complementarity-determining region (CDR) of the immunoglobulin molecule, wherein the heterologous peptide comprises two or more T cell epitopes as defined above.

As disclosed herein, a single inoculation of the H chain gene targeted to spleen lymphocytes is sufficient to initiate immunity (see Example I), establish immunologic memory (see Example III), and program the immune response predictably and reproducibly. Experiments in murine systems, in vitro and *in vivo,* demonstrate that the H chain polypeptides of the transgene associate with endogenous light chains (Example IV), and transgenic Ig are secreted in amounts between 15 and 30 ng/ml (Example I). The synthesis of transgenic Ig is followed by an immune response consisting of antibodies and T cells specific for antigenic determinants of transgenic Ig by day 5-7. The antibody response remains detectable almost indefinitely. Upon booster injection with an appropriate antigen, a typical secondary immune response is induced.

In its simplest form STI is reflected by a model in which plasmid DNA is injected directly into a lymphoid organ where it reaches follicles and within them, the B lymphocytes. In the context of the invention, STI can bye realized as an *ex vivo* process in which normal B lymphocytes are transfeceted *in vitro* and subsequently injected *in vivo* (Example IX). In either case, the B lymphocytes that uptake the foreign DNA coding for the transgene transcribe and translate the transgene into functional polypeptide chains. Assembled polypeptides form transgenic Ig carrying heterologous epitopes (antigenized transgenic Ig). Secreted transgenic Ig elicit an immune response by B lymphocytes against the antigenic determinants born on transgenic Ig. Transgenic Ig can also activate T cells. T cell determinant peptides are processed and presented either by B lymphocytes harboring the transgene (direct presentation) or by dendritic cells (DC) (secondary-priming). The process of immunity spreads rapidly to other secondary lymphoid organs through secreted transgenic Ig reaching the bloodstream and the lymphatic system (Example VI). As the response evolves in time, transgenic Ig alone or complexed with specific antibodies are trapped by follicular dendritic cells (FDC) and stored along the dendrites to be re-utilized during memory responses.

Secreted transgenic Ig can target APC via the Fc receptor for secondary antigen processing and presentation, hence acting as source of antigen peptides for lymphoid tissues distal from the site of initiation of immunity. From this it is easy to see how immunity can spread from the initial site. In fact, cells harboring the transgene do not colonize other lymphoid organs (see Example II). Transgenic Ig emigrate from the organ of inoculation and diffuses to other districts of the lymphoid system through the bloodstream and the lymphatics. There they can promote immunity de *novo.* Unlike conventional immunization systems, where antigen or antigen peptide in immunologic adjuvant activate T cells only in draining lymph nodes (Kearney et al., Immunity 1:327-339(1994), during STI, mobilization of activated T cells together with the diffusion of soluble transgenic Ig facilitate spreading of T cell immunity throughout the body (see Example VIII).

In transgenic Ig, B-cell epitopes are expressed with controlled geometry and spatial characteristics to approximate the shape of native antigens from which they derive. Since the antigen receptor on B lymphocytes recognizes antigens through their three-dimensional structure and binds establishing interactions over large sterically and electrostatically complementary areas, the expression of B cell epitopes in antibody loops induce antibodies cross-reactive with a native structure. Activation of T cells, on the other hand, requires that antigen be presented in the form of small peptides. As disclosed herein, T-cell peptides expressed in CDR loops are easily processed and presented in the context of major histo-compatibility complex (MHC) molecules (see Example VI and VII, and Zanetti et al., Immunol. Rev., 130:125-150 (1992); Zaghouani et al., Proc. Natl. Acad. Sci. USA, 92:631-635 (1995); Zaghouani et al., Science, 259:224-227 (1993); Billetta et al., Eur. J. Biochem., 25:776-783 (1995)). Thus, during somatic transgenesis, B cells harboring and synthesizing transgenic Ig become a self-renewing source of T cell peptides.

In addition to being formidable minifactories of proteins in mammals, B lymphocytes can also present antigen to T lymphocytes: (i) antigens internalized via their membrane Ig receptor (Lanzavecchia, Nature, 314:537-539 (1985)), and (ii) peptides of secretory proteins including their own Ig (Weiss and Bogen, Proc. Natl. Acad. Sci. USA 86:282-286 (1989); Billetta et al., Eur. J. Immunol. 25:776-783 (1995)). Because of these properties, B lymphocytes constitute an ideal substrate for strategies of gene targeting and immunization with plasmid DNA.

As disclosed herein in Example VI, cellular immune responses were analyzed *in vivo* after a single intraspleen inoculation of DNA coding for a 12 residue Th cell determinant associated with a 12 residue B cell epitope, a process termed somatic transgene immunization. As disclosed herein, CD4 T cells are readily activated and produce IL-2, IFN-γ and IL-4, characteristics of an uncommitted phenotype. Although originating in the spleen, T cell responsiveness was found to spread immediately and with similar characteristics to all lymph nodes in the body. A single inoculation was also effective in establishing long term immunologic memory as determined by limiting dilution analysis, with memory T cells displaying a cytokine profile different from primary effector T cells. These studies provide evidence that by initiating immunity directly in secondary lymphoid organs, one generates an immune response with characteristics that differ from those using vaccines of conventional DNA or protein in adjuvant administered in peripheral sites.

When a transgene coding for a strong Th (CD4) cell determinant is inoculated into mice, a vigorous CD4 T cell response is elicited (Gerloni et al., J. Immunol., 162:3782-3789 (1999)). The activation of Th cells is reproducible and always hallmarked by the concomitant production of large amounts of IL-2 and proportional amounts of IFN-_{Y} and IL-4. Conventional DNA immunization favors Th1 responses (Roman et al., *supra,* 1997; Chu et al., J. Exp. Med. 186:1623-1631 (1997)). STI activates uncommitted CD4 T cells.

When a transgene coding for a strong class I MHC-restricted T (CD8) cell determinant is inoculated into mice, a specific CD8 T response with protection was measured (see Example VII). The results disclosed herein indicate that STI serves as an endogenous source of T cell peptides and has fulfilled basic requirements for immunogenicity *in vivo.*

As disclosed herein, the plasmid DNA coding for an immunoglobulin heavy (H) chain gene used is under the control of tissue-specific promoter and enhancer elements (Banerji et al., Cell 33:729-740 (1983); Gillies et al., Cell 33:717-728 (1983); Grosschedl and Baltimore, Cell 41:885-897 (1985); Mason et al., Cell 41:479-487 (1985)).

The type of immunogenic stimulus offered by somatic transgene immunization can persist in the organism as long as B lymphocytes harboring the transgene live, synthesize and secrete transgenic Ig. The transgene can persist in the host throughout the life span of the host B cell to disappear when the B cell dies. This, together with the "depot effect" played by follicular dendritic cells, may be critical in the induction and maintenance of memory B cells whose half-life in the absence of antigen is estimated in the order of 2-3 weeks (Gray and Skarvall, Nature 336:70-73 (1988)).

The results described herein illustrate the use of STI to induce antigen-specific immunity agaisnt a microbial pathogen (see Example III). STI immunized against three repeats of the hydrophilic tetrapeptide sequence Asn-Ala-Asn-Pro (NANP), a B-cell epitope expressed on the surface of Plasmodium *falciparum* malaria sporozoites, engineered in the CDR3 of a H chain gene. This amino acid sequence is present in multiple tandem repeats in the central portion of the circumsporozoite (CS) protein (Zavala et al., Science 228:1436-1440 (1985)). Antibodies against this epitope develop in people living in endemic areas for malaria (Zavala et al., *supra,* 1985; Nardin et al., Science 206:597-601 (1979)) as well as in volunteers vaccinated with irradiated sporozoites (Clyde et al., Am. J. Med. Sci. 266:398-403 (1973); Calle et al., J. Immunol. 149:2695-2701 (1992); Egan et al., Am. J. Trop. Med. Hyg. 49:166-173 (1993)).

As disclosed herein in Example III, immunity against the human malaria parasite *Plasmodium falciparum* was induced using somatic transgene immunization. A single inoculation of plasmid DNA containing an immunoglobulin heavy chain gene coding in the CDR3 for three repeats of the sequence Asn-Ala-Asn-Pro (NANP), a B-cell epitope of *P. falciparum* sporozoites, induced antibodies against NANP in all mice.

The invention can be used to stimulate a T cell response such as a CD4 T cell response and/or a CD8 T cell response. Hypervariable loops of immunoglobulins (Ig) can be used to express discrete peptide sequences of antigens, antigenized antibodies (Zanetti, Nature, 355:466 (1992)). These can be the amino acid sequences of epitopes that induce specific responses in T lymphocytes, CD4⁺ and CD8⁺.

As disclosed herein in Example VI, cellular immune responses were analyzed *in vivo* after a single intraspleen inoculation of DNA coding for a 12 reside Th cell determinant associated with a 12 residue B cell epitope, a process termed somatic transgene immunization. As disclosed herein, CD4 T cells are readily activated and produce IL-2, IFN-_{Y} and IL-4, characteristics of an uncommitted phenotype (Th0). Although originating in the spleen, T cell responsiveness was found to spread immediately and with similar characteristics to all lymph nodes in the body. A single inoculation was also effective in establishing long term immunologic memory as determined by limiting dilution analysis, with memory T cells displaying a cytokine profile different from primary effector T cells.

These studies provide evidence that somatic transgene immunization is a useful method to induce Th cell responsiveness *in vivo.*

The invention is also useful for stimulating an antibody response in combination with a T cell response such as a CD4 T cell response. Such a combined response can be termed associative recognition. Inclusion of multiple epitopes from the same antigen or combination of epitopes with different immunogenic function in the same molecule can be used in nucleic acid molecules of the invention. For instance, the antibody response to protein antigens requires the cooperation between B cells and T helper (Th) cells (Mitchison, Eur. J. Immunol. 1:18-27 (1971)) with optimal conditions occurring when B and Th cells are specific for different determinants on the same molecule (associative recognition).

As disclosed herein, an antigenized antibody gene coding for two distinct 12 amino acid long peptides representing a B (Zavala et al., Science, 228:1436-1440 (1985)) and a Th (Munesinghe et al., *supra,* 1991; Nardin et al., Science 246:1603-1606 (1989) cell epitope of the circumsporozoite (CS) protein of *P. falciparum* malaria parasite were expressed and tested. Engineering of the CDR3 and the CDR2 of the same V_{H} domain did not significantly affect secretion *in vivo* of the antigenized antibody molecules. Mice inoculated into the spleen with this gene mounted an antibody response against the B cell epitope higher than mice receiving the gene coding for the B cell epitope only. In vitro studies established that the two epitope were independently immunogenic *in vivo* (see Example IV).

The invention can similarly by used for associative recognition to stimulate a Th/Th response. While the importance of associative (linked) recognition events in the development of an adaptive immune response are universally accepted, it is not known yet whether or not the same concept applies to a cooperative interaction between Th cell epitopes on the same molecule. Experiments using an antigenized antibody gene in the context of STI revealed that this is the case (see Figure 35 and Example X).

As disclosed herein, two Th cell epitope expressed in the CDR2 and CDR3 of the same gene, respectively, were independently immunogenic *in vivo* (Figure 36 and Example X).

The ability to manipulate Ig V region genes and express multiple heterologous peptides in the CDRs open new possibilities in the design of molecules of complex, predetermined antigen specificity and/or complementary immunogenic function, for example, B/Th, Th/Th or Th/CTL epitopes, depending on the desired effect, for vaccination purposes.

A key feature of STI is the establishment of persistent immunologic memory. Booster injection of the γ1NANP protein in adjuvant 6, 30 or 104 weeks after priming resulted in a *bona* fide anamnestic response. Specific memory also exists when mice were challenged with *P. falciparum* parasites 6 weeks after priming (see Example III).

As disclosed herein, a natural immunologic adjuvant, GM-CSF, was shown to increase the potency of immunization by STI (see Example V). GM-CSF given at priming as a DNA/GM-CSF chimeric vaccine enhances the magnitude of the anamnestic response irrespective of the form of antigen used subsequently in the booster immunization.

As disclosed herein, priming with an antigenized antibody /GM-CSF DNA vaccine enhances the magnitude of the anamnestic response against a defined dodecapeptide B cell determinant irrespective of the form of antigen used in the booster immunization (Example V). The results disclosed herein define a role for the activity of GM-CSF *in vivo* as a modulator of the immune response, including immunologic memory.

As disclosed herein a nucleic acid molecule of the invention can be targeted to a lymphoid cell. The lymphoid cell can be targeted *ex vivo*.

As disclosed herein (Example IX) a nucleic acid molecule of the invention can be administered *ex vivo.* For example, hematopoietic cells, including lymphoid cells, can be obtained from an individual or from an immunologically compatible individual, and a nucleic acid molecule of the invention can be administered to these cells *ex vivo.* Methods of administering nucleic acid molecules to cells *ex vivo* are well known in the art and include, for example, calcium phosphate precipitation and electroporation (see, for example, Sambrook et al., Molecular Cloning a Laboratory Manual Cold Spring Harbor Press (1989); Ausubel et al., Current Protocol in Molecular Biology, Wiley & Sons (1998)). A method of administering nucleic acid molecules to cells *ex vivo* is also described in Example X. These lymphoid cells, which now contain the nucleic acid molecule and express the encoded epitopes, can then be administered to an individual. The lymphoid cells expressing the epitopes can then stimulate an immune response.

Disclosed is treating a condition by administering a nucleic acid molecule of the invention, where the nucleic acid molecule is targeted to a hematopoietic cell. Disclosed is treating a condition, comprising administering a non-viral vector comprising a nucleic acid molecule comprising a B cell-specific expression element operationally linked to a nucleic acid sequence encoding a heterologous polypeptide, wherein the nucleic acid molecule is targeted to a B cell and expresses the heterologous polypeptide. Similarly, a T cell can be targeted with a non-viral vector containing a T cell-specific expression element operationally linked to a nucleic acid encoding a heterologous polypeptide. As used herein, a "non-viral vector" refers to a nucleic acid that can function as a vector but is not encapsulated in a virus or encoded in a viral genome. The administration of a nucleic acid molecule expressing an epitope to stimulate an immune response is useful for treating a condition as described above. The methods for treating a condition by targeting a hematopoietic cell can be used by targeting a B cell or T cell. The methods for treating a condition are particularly useful when a B cell is targeted.

Disclosed is treating a condition by administering a nucleic acid molecule comprising a hematopoietic cell-specific expression element operationally linked to a nucleic acid molecule encoding one or more heterologous polypeptides, where the nucleic acid molecule is targeted to a hematopoietic cell. The targeted hematopoietic cells serve to express a heterologous polypeptide to treat a condition. The methods are advantageous for administering a therapeutic polypeptide to treat a condition. The methods can be used, for example, to express a hormone, cytokine, clotting factor or immunoglobulin. For example, if an individual has a condition for which an increase in expression of a hormone or cytokine would be beneficial, such an individual can be treated by administration of a nucleic acid molecule expressing a hormone or cyotokine polypeptide. For example, an individual having a condition characterized by immunodeficiency can be treated by administering a cytokine such as IL-2 or INF-_{Y}, or other cytokine, as disclosed herein, or by administering an immunoglobulin. Similarly, an individual suffering from a condition such as hemophelia can be treated, for example, by administering a nucleic acid molecule encoding a clotting factor such as factor VIII or factor IX. One skilled in the art can readily determine an appropriate polypeptide to express for treating a given condition.

The methods can be used to treat a condition by expressing a wide variety of disease-associated gene products of interest, which can be employed to treat or prevent the disease of interest. For example, and by way of illustration only, the genes can encode enzymes, hormones, cytokines, antigens, antibodies, clotting factors, anti-sense RNA, regulatory proteins, ribozymes, fusion proteins and the like. The methods can thus be used to supply a therapeutic protein such as Factor VIII, Factor IX, Factor VII, erythropoietin (U.S. Patent No. 4,703,008), alpha-1-antitrypsin, calcitonin, growth hormone, insulin, low density lipoprotein, apolipoprotein E, IL-2 receptor and its antagonists, superoxide dismutase, immune response modifiers, parathyroid hormone, the interferons (IFN alpha, beta or gamma), nerve growth factors, glucocerebrosidase, colony stimulating factor, interleukins (IL) 1 to 15, granulocyte colony stimulating factor (G-CSF), granulocyte, macrophage-colony stimulating factor (GM-CSF), macrophage-colony stimulating factor (M-CFS), fibroblast growth factor (FGF), platelet-derived growth factor (PDGF), adenosine deaminase, insulin-like growth factors (IGF-1 and IGF-2), megakaryocyte promoting ligand (MPL, or thrombopoietin). The therapeutic polypeptides can be useful, for example, for the treatment and prevention of genetic disorders such as coagulation factor disorders, glycogen storage disease, and alpha-1-antitrypsin deficiency. The methods can also be used to express ligands of adhesion molecules such as integrins, for example, to block adhesion function such as angiogenesis.

Disclosed are pharmaceutical compositions comprising a pharmaceutically acceptable carrier and a nucleic acid molecule of the invention. The invention can therefore utilize pharmaceutical composition comprising a nucleic acid molecule of the invention encoding the two or more epitopes Pharmaceutically acceptable carriers are well known in the art and include aqueous or non-aqueous solutions, suspensions and emulsions, including physiologically buffered saline, alcohol/aqueous solutions or other solvents or vehicles such as glycols, glycerol, oils such as olive oil or injectable organic esters.

A pharmaceutically acceptable carrier can contain physiologically acceptable compounds that act, for example, to stabilize the nucleic acid molecules to be administered or increase the absorption of the nucleic acid molecules. Such physiologically acceptable compounds include, for example, carbohydrates, such as glucose, sucrose or dextrans, antioxidants such as ascorbic acid or glutathione, chelating agents, low molecular weight polypeptides, antimicrobial agents, inert gases or other stabilizers or excipients. Nucleic acid molecules can additionally be complexed with other components such as peptides, polypeptides and carbohydrates. Nucleic acid molecules can also be complexed to particles or beads that can be administered to an individual, for example, using a vaccine gun. One skilled in the art would know that the choice of a pharmaceutically acceptable carrier, including a physiologically acceptable compound, depends, for example, on the route of administration of the expression vector. As described above, the route of administration can be by direct injection into a secondary lymphoid tissue.

Administration can also be at a site other than the lymphoid tissue but that targets the lymphoid tissue. An invention nucleic acid can be administered systemically via the blood, for example, by intravenous injection and targeted to a lymphoid cell in a lymphoid tissue. Nasal administration or oral administration can also be used. For example, a vector in the form of a bacterium containing an invention nucleic acid can be administered orally and will target to Payer's patches.

The B and T cells targeted in both *in vivo* and *ex vivo* methods of the invention are normal cells, that is, non-tumor cells. The cells can be untreated and unstimulated.

The following examples are intended to illustrate but not limit the present invention.

### EXAMPLE I

### Somatic Transgene Immunization with DNA Encoding an Immunoglobulin Heavy Chain

This example describes immunization with plasmid DNA by direct injection into the spleen.

The methods for preparation of plasmid DNA and immunization by injection into the spleen are as described in (Gerloni et al., DNA Cell Biol. 16:611-625 (1997)), Figure 1.

Mice were inoculated with 100 µg of plasmid DNA per inoculation. All DNA inoculations were done in the absence of immunological adjuvants. Four basic routes of inoculation were used. a) Intramuscular. The plasmid DNA was injected in the quadriceps in 30 µl volume in sterile saline. Thereafter, mice received three booster injections at weekly intervals for a total of four injections. b) Subcutaneous. The plasmid DNA was injected in the back in 25-50 µl volume of sterile saline. Thereafter, mice received three booster injections at weekly intervals for a total of four injections. c) Intravenous. The plasmid DNA was injected in 50-100 µl volume of sterile saline solution via the tail vein. Thereafter, mice received three booster injections at weekly intervals for a total of four injections. d) Intraspleen. The plasmid DNA was injected in 30 µl volume of sterile saline solution.

Mice were immunized with affinity-purified _{Y}1WT protein adsorbed on alum (50 µg per mouse) intraperitoneally. Mice that were boosted with the γ1WT protein received 50 µg of the protein emulsified in incomplete Freunds' adjuvant subcutaneously.

The presence of γ1WT H chain transgene polypeptide in the serum of mice was detected by ELISA capture assay (Billetta and Zanetti, Immuno. Methods, 1:41-51 (1992)). Briefly, 1:10 dilution of individual mouse sera in PBSA were incubated on 96-well plate coated with a goat antibody to human γ-globulin (10 µg/ml). The concentration of the immunoglobulin H chain transgene product in the serum was calculated by plotting the O.D. values against a standard curve constructed with known amount of human γ-globulins.

For extraction of genomic DNA from spleen tissue and genomic DNA sequencing, spleens were harvested 17 days after DNA inoculation, frozen at -170°C and the cells were prepared by tissue grinding in liquid nitrogen. Typically the genomic DNA was extracted from 10 mg of spleen tissue using the QIAamp Tissue Kit (Qiagen Inc.; Chatsworth CA). Two specific primers, TTATTGAGAATAGAGGACATCTG and ATGCTCAGAAAACTCCATAAC for the murine V_{H}62 were used to amplify by PCR a segment of 520 bp from genomic DNA. The PCR conditions were as follows: 45 sec at 94°C, 45 sec at 54°C and 45 sec at 72°C for 30 times. The PCR products were cloned in pGEM-T vector (Promega; Madison WI). Six clones from the genomic DNA of the spleen inoculated 17 days earlier and four clones from the genomic DNA of tranfectoma B cells (Sollazzo et al., *supra,* 1989) were sequenced on both strands by dideoxy termination method with Sequenase 2.0 DNA sequencing kit (USB; Cleveland OH) using two primers, AACAGTATTCTTTCTTTGCAGG and TTATTGAGAATAGAGGACATCTG, annealing 10 bp before the first codon of the FR1 and at the 3' end of the FR4, respectively.

Mice were immunized via the intrasplenic route and by comparison via other routes of inoculation, for example, intramuscular, subcutaneous, and intravenous. Table 1 shows the anti-immunoglobulin response determined by an ELISA method in mice inoculated through the various routes with the number of injections in each case. A marked antibody response was seen only in mice inoculated once via the intrasplenic route (group I). Mice inoculated once via the intrasplenic route and boosted intravenously three times (group V) also responded but because the three additional intravenous injections yielded a substantially similar antibody titer, a logical conclusion is that the antibody response seen in group V reflects mainly the effect of intraspleen inoculation. The subcutaneous route yielded a weak response in two mice only (group III). No antibody response was detected in mice inoculated four times intramuscularly or intravenously (groups II and IV). Thus, the use of an immunoglobulin H chain gene under the control of tissue specific regulatory elements yielded immunity only after intraspleen inoculation.

**Table 1. Production of Antibodies Reacting with the γ1WT Protein in C57B1/6 Mice Inoculated with γ1WT DNA: Effect of the Route of Inoculation**

| Group | Route of Inoculation | Injections (no.) | Mice (no.) | Responders (no.) | Antibody titer^{a} (log) |
|---|---|---|---|---|---|
| I | i.s. | 1 | 4 | 4/4 | 3.1 ± 0.4 |
| II | i.m. | 4 | 4 | 0/4 | ≤ 2.3^{b} |
| III | s.c | 4 | 4 | 2/4 | 2.6 |
| IV | i.v | 4 | 4 | 0/4 | ≤ 2.3 |
| V | i.s + i.v | 1+3 | 4 | 4/4 | 3.2 ± 0.3 |

| | | | | | |
|---|---|---|---|---|---|
| ^{a}Values of antibody titer were measured and calculated on sera collected 21 days after the first inoculation. ^{b}The preinoculation value of a large pool of mice was 2.3 (log). The end-point positive serum dilution on which the titer was calculated was an OD value (A₄₉₂) ≥0.200. | | | | | |

The H chain transgene product could not be detected beyond day 26 possibly due to the formation of immune complexes with anti-immunoglobulin antibodies. Thus, inoculation of an immunoglobulin H chain DNA via the intrasplenic route yielded a measurable secretion of the transgene immunoglobulin product in 100 percent of cases until the day 26.

**Table 2. Detection of the Transgene Immunoglobulin Product in the Serum of C57B1/6 Mice After a Single Intraspleen Inoculation of DNA**

| | | | | **Production (ng/ml)** | |
|---|---|---|---|---|---|
| Experiment number | Material inoculated | Mice (no.) | Producers (no.) | Mean ±±SD | Range |
| 1 | γ1WT | 14 | 14/14 | 7.3 ± 7.6^{a} | 1.0-21.1 |
| 2 | γ1WT | 7 | 7/7 | 32.1 ± 22.7 | 10.3-72 |
| 3 | γ1WT | 9 | 9/9 | 9.3 ± 5.1 | 5.1-15 |
| 4 | pSV2neo | 7 | 0/7 | -- | -- |
| 5 | Saline | 3 | 0/3 | -- | -- |

Values of transgene product in the serum represented correspond to the day of maximal detection for each individual mouse. Determination of circulating transgene immunoglobulins was done as described above. The experiments and the ELISA were done independently and at different times.

DNA sequencing was used to determine whether persistence *in vivo* in the host cell DNA would cause the transgene to undergo somatic mutation. Because somatic mutation is property of the VDJ coding region (Griffiths et al., Nature 312:271-275 (1984)), this region only was characterized. The VDJ coding region (520 bp) was amplified from genomic DNA using specific primers as described above. Altogether, sequencing was done in six clones from genomic DNA of an inoculated spleen and four clones from genomic DNA of transfectoma B cells which served as reference. The nucleotide sequence of the six clones showed no mutation with the exception of a single conservative (C to T) mutation in framework 3 in clone SP7. A single (C to T) mutation was also observed in framework 2 in clone TR38 from transfectoma B cells DNA (Figure 2). Thus, the VDJ coding region of the transgene retrieved in an integrated form 17 days after intraspleen inoculation did not show evidence of hypermutation. Thus, a lack of somatic mutation in the transgene *in vivo* was observed.

These results demonstrate that a nucleic acid molecule can be administered to a lymphoid tissue, the spleen, to elicit an immune response.

### EXAMPLE II

### In vivo Role of B Lymphocytes in Somatic Transgene Immunization

This example describes the role of B lymphocytes in somatic transgene immunization.

The preparation of plasmids and immunization are described below (Xiong et al., Proc. Natl. Acad. Sci. USA 94:6352-6357 (1997)).

Plasmid γ1NANP (Sollazzo et al., Protein Eng., 4:215-220 (1990a)) (Figure 1) carries a chimeric H chain gene in which a productively rearranged murine V region gene is joined to a human _{Y}1 C region gene. The V region of this H chain gene was modified in the third complementarity determining region (CDR3) by introduction of the nucleotide sequence coding for three Asn-Ala-Asn-Pro repeats (Sollazzo et al., *supra,* 1990a). The promoter and enhancer elements in this plasmid are those constitutively existing in Ig H chain genes and have been described previously (Sollazzo et al., *supra,* 1989). Plasmid pSVneo is the original plasmid vector that lacks the murine V region and the human γ1 C region genes (Mulligan and Berg, Proc. Natl. Acad. Sci. USA, 78:2072-2076 (1981)).

Antibodies to γ1NANP or synthetic petide (NANP)n were detected on 96-well polyvinyl microtiter plates coated with affinity-purified antibody γ1NANP (2.5 µg/ml) or synthetic peptide(5 µg/ml). Sera were diluted in PBSA. The bound antibodies were revealed using a HP-conjugated goat antibody to mouse γ-globulins absorbed with human γ-globulins (Pierce; St. Louis MO). The bound peroxidase was revealed by adding o-phenylenediamine dihydrochloride and H₂O₂. Tests were done in duplicate. The presence of transgene H chain immunoglobulins in the serum was detected using a capture ELISA (see Example I; Billetta and Zanetti, *supra,* 1992).

For DNA sequencing, a 566 bp DNA fragment containing the whole VDJ coding region was amplified from splenic genomic DNA using two primers (pCL and pCD) specific for the rearranged murine V_{H}. This fragment was subcloned into the pGEM-T vector (Promega; Madison WI). The plasmid DNA was extracted from transformed DH5γ *Escherichia coli* and sequenced by dideoxy termination method with SEQUENASE 2.0 DNA Sequencing Kit (USB; Cleveland, OH) using two primers (pSE and pCD) annealing in front of the FR1 and at the end of FR4 from opposite directions.

For fluorescence-activated cell sorting (FACS), spleen cells were prepared by grinding the spleen tissue harvested 15, 21 and 28 days after inoculation, or from naive mice. The cell suspension was washed twice with 0.5% PBSA and the red blood cells were removed by treatment with lysing buffer (Sigma; St. Louis MO). The lymphocytes were differentially stained with phycoerythrin (PE)-conjugated rat anti-mouse Ly-5 (B-220) Pan B-cell (Caltag; San Francisco CA), fluorescein isothiocyanate (FITC)-conjugated rat anti-mouse CD4 (Caltag) and FITC-conjugated rat anti-mouse CD8 (Caltag) for 20 min at 4°C. The cell suspension was washed twice in 0.5% PBSA and resuspended at the concentration of 5×10⁶ cells/ml in DMEM (Irvine Scientific; Irvine CA). The cells were sorted on a FACSTAR (Becton & Dickinson; San Jose CA). Genomic DNA was extracted from 1×10⁶ B or T lymphocytes using the QIAAMP Blood kit (Qiagen). The DNA fragments were amplified by PCR and run on a 1% agarose gel. They were subsequently transferred to a nylon membrane for Southern blot hybridization using the (³²P)-labeled pNAD oligonucleotide.

To demonstrate that B lymphocytes are the target cell population *in vivo* for the transgene, the following experiment was performed. Starting from the second week after plasmid DNA inoculation, splenic B and T lymphocytes were isolated to a high degree of purity (97-99%) by FACS sorting (Figure 3). The genomic DNA was extracted from the two cell populations and amplified by PCR. PCR was performed with a total of four sets of primers, pCL and pCD; pSE and pNAD; pNEL and pNED; and pγA1 and pγA2. pCL γfrom -107nt to -85nt: 5'-TTATTGAGAATAGAGGACATCTG-3'; and pCD γfrom 459nt to 439nt: 5'-ATGCTCATAAAACTCCATAAC-3'; were used to amplify the whole VDJ region of the transgene. pSE γfrom -32nt to -11nt: 5'-AACAGTATTCTTTCTTTGCAGC-3'; and pNAD γfrom 352nt to 333nt: 5'-GAGAGTAGGGTACTGGGTTT-3'; were specific for amplification of the genetic marker, (NANP)₃ in CDR3. pNEL γfrom 169nt to 189nt: 5'-AGCACCTACTATCCAGACACT-3'; and pNED γfrom 366nt to 346nt:
5'-GTAGTCCATACCATGAGAGTA-3'; were the inner primers for nested PCR. pγA1 γfrom 184nt to 201nt:
5'-TGGGCCGCCCTAGTCACC-3'; and pγA2 γfrom 427nt to 408nt: 5'-CGTTTGGCCTTAGGGTTCAG-3'; were designed to amplify the murine β-actin gene according to the sequence indicated in (Harris et al., Gene 112:265-266 (1992)). The PCR consisted of 30 cycles at 94°C for 45 sec, 58°C for 45 sec, and 72°C for 45 sec; 0.3 µM each primer; 0.2 mM each deoxynucleotide; 1.5 mM MgCl₂ in 20 mM Tris-HCl, pH 8.4 and 50 mM KCl; and 1 unit of Taq DNA polymerase (Gibco BRL; Gaithersburg MD). PCR products for Southern blot analysis were resolved in 1% w/v agarose gel and blotted onto HYBOND-N nylon membrane (Amersham; Cleveland, OH). The membranes were hybridized with the oligonucleotide pNAD labeled using T4 polynucleotide kinase forward reaction in presence of (γ³²P-ATP). At the 15 day time point, distinct amplification products were readily detectable in both B and T lymphocytes. However, at both the 21 and 28 day time points, specific amplification was observed only in B cells. Southern blot hybridization confirmed the specificity of the amplification products. These results suggested that B lymphocytes in the spleen are the target cell population in which the transgene persists for a long time.

The transgene was sequenced from genomic DNA. The transgene VDJ region was amplified from splenic genomic DNA, subcloned and sequenced by the dideoxy termination method. No evidence of hypermutation was found in the VDJ region of the transgene even after 3 months *in vivo* (Table 3).

**Table 3. Lack of transgene mutations in PCR-generated clones from splenic genomic DNA.**

| Time (wk) | No. of clones sequenced | No. of clones mutated | No. of nucleotides mutated | Rate of mutation* (%) |
|---|---|---|---|---|
| 2 | 6 | 1/6 | 1** | 2.9 x 10⁻⁴ |
| 4 | 3 | 0/3 | 0 | |
| 12 | 3 | 0/3 | 0 | |

| | | | | |
|---|---|---|---|---|
| * Number of mutations per total number of base pairs sequences. ** A silent (C to T) mutation in FR3. | | | | |

These results demonstrate that *in vivo* inoculation with plasmid DNA resulted in expression of the transgene in B cells of the spleen for at least three months.

### EXAMPLE III

### Immunity to a Microbial Pathogen by Somatic Transgene Immunization

This example describes administration of a nucleic acid molecule encoding a B-cell epitope of *P. falciparum* malaria parasite to induce an immune response against the parasite antigen.

The protocols used are described below (Gerloni et al., Nature Biotech. 15:876-881 (1997)).

_{Y}1NANP and pSV2Neo are described in Figure 1 and Example II. The detection of antibodies to synthetic peptide (NANP)n was done as described in Example II. Other substrates included the γ1NANP protein and R32LR antigen.

Sera diluted 1:50 were assayed for immunofluoresence reactivity with air dried *P. falciparum* sporozoites at various dilutions (1:25 to 1:800). The assays were performed as previously described (Wirtz et al., Exp. Parasitol., 63:166-172 (1987)). Fluorescence intensity was graded from 0 to 4+, with 0 indicating no fluorescence detectable and 4+ indicating intense fluorescence over the entire surface of the sporozoites. Sample with β+ fluorescence intensity were considered positive.

Mice were inoculated with 100 µg of plasmid DNA in 30 µl of sterile saline solution intraspleen as detailed under Example I. In the experiment described in Table 4 mice, were boosted with 100 µg of plasmid DNA γ1NANP in saline administered intravenously via the tail vein.

**Table 4. Titers (log₁₀) if antibodies reacting with NANP peptide after priming and booster immunizations.**

| Group | Priming* | Booster | No. of mice | Primary immune response (days) | | | | Secondary immune response (days) | | |
|---|---|---|---|---|---|---|---|---|---|---|
| | | | | **0** | **14** | **28** | **53** | **200** | **214** | **228** |
| I | γ1NANP DNA | γ1NANP DNA | 4 | ≤2.3 | 2.6 | 2.8±0.2 | 2.8±0.2 | 2.9±0 | 2.9±0 | 2.9±0 |
| II | γ1NANP DNA | γ1NANP protein | 4 | ≤2.3 | 2.6 | 2.9 | 2.8±0.2 | 3±0.2 | 3.6±0.3 | 3.7±0.4 |
| III | pSVneo DNA | γ1NANP protein | 4 | ≤2.3 | ≤2.3 | ≤2.3 | ≤2.3 | ≤2.3 | ≤2.3 | ≤2.3 |
| IV | γ1NANP protein | γ1NANP protein | 4 | ≤2.3 | ≤2.3 | ≤2.3 | ≤2.3 | 2.4±0.3 | 2.5±0.4 | 2.6±0.6 |
| V | OVA protein | OVA protein | 4 | ≤2.3 | ≤2.3 | ≤2.3 | ≤2.3 | ≤2.3 | ≤2.3 | ≤2.3 |

| | | | | | | | | | | |
|---|---|---|---|---|---|---|---|---|---|---|
| * All priming injections were done through the intraspleen route. Booster injections were done on day 200. In all but one group (group 1, which was done intravenously) booster injections were done subcutaneously. | | | | | | | | | | |

Mice were inoculated i.s. with affinity-purified γ1NANP protein in sterile saline solution. The surgical procedures were as described above. Mice were immunized with affinity-purified γ1NANP protein emulsified in complete Freunds' adjuvant (50 µg per mouse) subcutaneously. Mice that were boosted with the γ1NANP protein received 50 µg of the protein emulsified in incomplete Freunds' adjuvant subcutaneously or 50 µg of the protein adsorbed on alum intraperitoneally. 10⁵ irradiated sporozoites in incomplete DMEM were injected intraperitoneally in a 0.4 ml volume. Mice were bled via the retro-orbital route.

Inoculation of plasmid γ1NANP DNA γ1NANP induces a primary response against the peptide NANP. Table 4 summarizes the ELISA antibody responses in which anti-NANP peptide antibodies were found in mice primed with the H chain transgene (γ1NANP DNA) (groups I and II). Antibodies appeared by day 14 and reached a plateau by day 28 (log 2.8)(Table 4). Circulating antibodies persisted through day 200 when mice received a booster injection. The antibody response against the intact antigenized antibody γ1NANP paralleled the response against the synthetic peptide. Mice inoculated intrasplenically with 50 µg of the γ1NANP protein (group IV) failed to mount any measurable anti-peptide response, although a modest elevation in titer against the intact _{Y}1NANP antibody was measured. Control groups injected with either the pSVneo plasmid or with ovalbumin failed to develop any antibody response above background titers higher than the pre-immunization values. No binding was observed when the same sera were tested on the synthetic peptide DENGNYPLQC used as a control.

Memory response against the NANP peptide was induced by γ1NANP DNA. A single intrasplenic inoculation of plasmid γ1NANP DNA γ1NANP was sufficient to induce immunologic memory against the (NANP)3 peptide expressed in the CDR3 of the H chain transgene. Table 4 shows the secondary anti-peptide response following a subcutaneous booster injection of the γ1NANP protein in incomplete Freunds' adjuvant (groups II and IV). The antibody titer against the synthetic NANP peptide rose in all animals in group II, and paralleled the response against the intact γ1NANP protein. In contrast, no anamnestic response occurred in mice boosted with a second intravenous injection of γ1NANP DNA (group I) perhaps because of the rapid degradation of plasmid DNA by plasma DNAses. The antibody response in mice primed by i.s. inoculation with soluble γ1NANP protein and boosted with γ1NANP protein subcutaneously (group IV) was similar to that seen with primary immunizations using the recombinant protein alone. No antibody responses against NANP were detected in control mice (groups III and V).

Immunization with _{Y}1NANP DNA induced immunologic memory response against *P. falciparum* sporozoites. To verify whether somatic transgene immunization could prime for immunologic memory upon encounter with the native CS protein of the parasite, mice were boosted by a single injection of *P. falciparum* sporozoites. The resulting antibody response was measured by ELISA. For comparison, mice were divided into two groups. One group was primed i.s. with plasmid DNA γ1NANP (or its control γ1WT). A second group was primed subcutaneously with antigenized antibody _{Y}1NANP in complete Freunds' adjuvant. Forty-five days after priming, mice were boosted with a single intraperitoneal injection of 10⁵ *P. falciparum* sporozoites or with antigenized antibody γ1NANP in incomplete Freunds' adjuvant by subcutaneous injections. Control groups included mice primed with plasmid γ1WT DNA or saline, and subsequently boosted with sporozoites. Mice primed with γ1NANP DNA and boosted with sporozoites (Figure 4) mounted a secondary response against NANP that was absent in mice primed with control plasmid DNA or with saline alone. Moreover, the anamnestic responses to sporozoites were greater in mice primed with γ1NANP DNA than in mice primed with the antigenized antibody γ1NANP in complete Freunds' adjuvant (CFA)(Figure 4A and 4C). Similar results were obtained when the sera were tested by ELISA on recombinant R32LR as capture antigen (Figure 4B and 4D).

These sera also reacted strongly with the surface of air-dried sporozoites by indirect immunofluoresence assay (Table 5), confirming that the DNA-immunized mice had been primed with a B cell epitope with a conformation that was substantially similar to that present on the surface of the target pathogen.

**Table 5. Antibodies reacting with Plasmodium falciparum sporozoites by IFA.**

| **Priming*** | **Booster*** | **IFA reactivity** |
|---|---|---|
| | | Titer^{γ} |
| γ1NANP DNA | -- | 25 |
| γ1NANP DNA | Sporozoite | 400 |
| γ1NANP DNA | γ1NANP protein | 50 |
| | | |
| γ1NANP protein | -- | 0 |
| γ1NANP protein | Sporozoite | 50 |
| γ1NANP protein | γ1NANP protein | 800 |

| | | |
|---|---|---|
| *Priming and booster injections were as described above. Sera were tested as pools of four mice each. Values shown represent the reciprocal of the last positive dilution. | | |

These results demonstrate that immunity to a microbial pathogen, *P. falciparum,* can be induced by administration of a nucleic acid molecule encoding a *P. falciparum* epitope.

### EXAMPLE IV

### Engineering Vaccines with Heterologous B and T Cell Epitopes Using Immunoglobulin Genes

This example describes the insertion of heterologous B and T cell epitopes into the CDRs of an immunoglobulin to enhance the immunologic response when administered as plasmid DNA.

The experimental procedures are described below (Xiong et al., Nature Biotechnology, 15:882-886 (1997)).

Plasmid γ1NV² NA³ was engineered as described below. The EcoRI fragment of the productively rearranged murine VH (2.3 Kb) was cloned in vector pBluescript II KS to yield plasmid pVH. Site-directed mutagenesis was performed using two 21mer oligonucleotide primers, one (5'-CAAGAAAGG**TAC**CCTACTCTC-3') annealing in CDR3 to introduce 3bp (TAC, in bold) for the creation of an Asp718 site, and another (5'-AGTAATGG**CCA**TGGTAGCACC-3') annealing in CDR2 to introduce 3bp (CCA, in bold) for the creation of a NcoI site. These primers were annealed to the uracylated, complementary strand of pVH and the mutant strands were synthsized and ligated in the presence of T4 DNA polymerase and ligase. Plasmid pVH-TAC/CCA, containing two unique sites, one in CDR3 (Asp718) and the other in CDR2 (NcoI), was obtained after transformation, screening of individual colonies and confirmation by DNA sequencing (SEQUENASE 2.0 DNA Sequencing Kit; USB; Cleveland OH). A pair of complementary oligonucleotides, 5'-GTACCCAATGCAAACCCAAATGCAAACCCAAATGCAAACCCA-3' (sense) and 5'-GTACTGGGTTTGCATTTGGGTTTGCATTTGGGTTTGCATTGG-3' (antisense) coding for the (NANP)3 sequence was synthesized, annealed and cloned in the Asp718 site. A pair of complementary oligonucleotides 5'-CATGGTAATGCAAACCCAAATGTAGATCCCAATGCCAACCCA-3' (sense) and 5'-CATGTGGGTTGGCATTGGGATCTACATTTGGGTTTGCATTAC-3' (antisense) coding for the NANPNVDPNANP sequence was similarly cloned into the NcoI site. The insertions and the proper orientation were verified by dideoxy sequencing (SEQUENCASE 2.0 DNA Sequencing Kit; USB). The 2.3Kb EcoRI fragment carrying the engineered CDR3 and CDR2 was then subcloned in the expression vector pNγ1 (Sollazzo et al., *supra,* 1989) upstream from the human γ1 constant (C) region using the unique EcoRI site to yield plasmid γ1NV² NA³. Plasmid γ1NANP carries a productively-rearranged murine V region gene in which only the CDR3 was modified by introducing the nucleotide sequence coding for three NANP repeats (Sollazzo et al., *supra,* 1990a). The promoter and enhancer elements in these plasmids are those constitutively existing in Ig H chain genes (Sollazzo et al., *supra,* 1989).

The recombinant antibodies γ1WT and γ1NANP were produced and purified as described previously (Billetta and Zanetti, supra, 1992; Sollazzo et al., *supra,* 1989). Detection of 6 and 8 light chains in circulating transgene H chain Ig was done as follows. Briefly, serum transgene H chain Ig were captured on 96-well plates coated with goat antibody to human IgGl (10 µg/ml) by incubation overnight at 4°C. The presence of murine light chains was assessed using a 1:2000 dilution of HP-conjugated goat antibodies to murine 6 or 8 light chains adsorbed with human Ig (Caltag; San Francisco CA). The assay was continued as described above. Tests were done in duplicate.

The engineering of two distinct epitopes in the same Ig V region gene was performed in the CDR3 and the CDR2 which contain a Asp718 (Sollazzo et al., Prot. Engineer., 3:531-539 (1990b)) and NcoI site, respectively. In the expressed proteins, both CDRs are loops interconnecting β-strands on the same β-sheet of the V domain. A modification of these two CDRs was expected to be compatible with proper VH/VL scaffolding, whereas engineering of the CDR1, which connects two different sheets of the V domain, could result in misfolding of the polypeptide. The B cell epitope used consisted of three repeats of the tetrapeptide Asn-Ala-Asn-Pro (NANP) from the CS antigen of *P*. *falciparum* parasite (Zavala et al., *supra,* 1985).

The Th cell epitope used is the peptide Asn-Ala-Asn-Pro-Asn-Val-Asp-Pro-Asn-Ala-Asn-Pro (NANPNVDPNANP), a conserved peptide sequence located in the 5' region of the CS antigen of *P. falciparum.* This peptide is recognized by immune human CD4* T lymphocytes (Nardin, et al., Science 246:1603-1606 (1989), is immunogenic for several MHC haplotypes in the mouse (Munesinghe et al., *supra,* 1991) and has been included in multiple-antigen-peptide vaccines for malaria.

The CDR3 and CDR2 of pVH were engineered as illustrated in Figure 5. The 2.3 Kb EcoRI DNA fragment carrying a productively-rearranged murine V_{H} cloned into pBluescript(pVH) was modified by oligonucleotide site-directed mutagenesis to introduce two unique cloning sites, Asp 718 site in CDR3 (Sollazzo et al., *supra,* 1990a) and NcoI in CDR2 (pVH-TAC/CCA). A pair of complementary synthetic oligonucleotides coding for three NANP repeats was cloned into the Asp 718 site whereas the pair coding for the NANPNVDPNANP sequence was cloned into the NcoI site of pVH-TAC/CCA. Nucleotide insertion and the correct orientation were checked by PCR and confirmed by sequencing (Figure 5A). The engineered 2.3 Kb EcoRI fragment was then cloned into the unique EcoRI site of the expression vector pNγ1 to yield plasmid γ1NV²NA³ (Figure 5B). The V region gene of plasmid γ1NV²NA³ codes, therefore, for two distinct epitopes of the CS antigen, one in CDR3 and the other in CDR2.

*In vivo* expression of transgene H chain antibodies was determined. As described in Example I, following intraspleen inoculation of plasmid DNA coding an Ig H chain gene, transgenic Ig were invariably detected in the circulation in amounts ranging between 15 and 30 ng/ml 10. Similar amounts were detected in mice inoculated with the antigenized H chain gene coding for the NANP epitope in CDR3 (see Example III). Mice inoculated with plasmid γ1NV²NA³ secreted transgene H chain Ig in amounts comparable to those secreted by mice inoculated with plasmid DNA γ1NANP (29.4 vs. 33.3 ng/ml). These results indicate that the modifications in the two CDR loops did not impact folding and secretion of transgene H chain Ig associated with endogenous light chains. This also suggests that transgene H chains with insertion of heterologous peptides in two CDRs are handled *in vivo* as conventional Ig H chain genes.

The immunogenicity of transgene H chain Ig carrying the two heterologous epitopes was analyzed by direct intraspleen inoculation of plasmid γ1NV²NA³. Mice inoculated with plasmid γ1NANP served as a control. Mice of both groups produced anti-(NANP)3 antibodies, indicating that in both instances, the CDR3 loops were immunogenic (Figure 6). However, the anti-NANP response in mice inoculated with plasmid γ1NV²NA³ was higher than in mice inoculated with plasmid γ1NANP (Figure 6A versus 6B). Whereas mice inoculated with plasmid γ1NV²NA³ produced antibodies reactive against both (NANP)3 and NANPNVDPNANP peptides (Figure 6B and 6D), mice inoculated with plasmid γ1NANP produced antibodies against (NANP)3 only (Figure 6A and 6C). Because antibodies to (NANP)3 do not cross-react with NANPNVDPNANP, mice inoculated with plasmid γ1NV²NA³ produced two distinct populations of antibodies, one against the (NANP)3 peptide in CDR3 and the other against the NANPNVDPNANP peptide in CDR2.

These results demonstrate that the two engineered CDRs were independently immunogenic *in vivo* and that the presence of the Th cell determinant in CDR2 enhanced the production of antibodies against the B cell epitope in CDR3.

### EXAMPLE V

### Immunological Memory After Somatic Transgene Immunization is Positively Affected by Priming with GM-CSF

This example describes enhanced immunological memory when an administered nucleic acid molecule is primed with GM-CSF.

The protocols used are described below (Gerloni et al., Eur. J. Immunol. 28:1832-1838 (1998)).

Plasmid γ1NANP/GM-CSF (DNA/GM-CSF) was contructed from plasmid γ1NANP (Example II) by cloning the murine GM-CSF coding sequence from plasmid p3159 at the 3' end of the CH3 domain of the constant through a Gly-Gly linker (Tao et al., Nature, 362:755-758 (1993)).

DNA vaccination consisted of a single intrasplenic inoculation of 100 µg of plasmid DNA in 30 µl of sterile saline solution as described in Example I. Mice immunized with the affinity-purified γ1NANP protein received a subcutaneous injection of the protein (50 µg/mouse) in complete Freunds' adjuvant (CFA). Booster injections consisted of either a single subcutaneous injection of affinity-purified γ1NANP protein (50 µg per mouse) emulsified in incomplete Freunds' adjuvant (IFA), or 10⁵ irradiated *P*. *falciparum* sporozoites injected intraperitoneally in a 0.4 ml of Dulbecco minimal essential medium. Sporozoites were produced in *Anopheles* freeborni mosquitos infected as described (Wirtz et al., supra, 1987).

Antibodies to synthetic peptide (NANP)n and γ1NANP were done as in Example II. The isotype of antibodies was determined using goat antibodies specific for the murine IgM and IgGl classes (Caltag; San Francisco CA) (see Example III).

GM-CSF heightens the anamnestic response induced by antigenized antibody in IFA. The anti-NANP response was measured in mice primed with DNA/GM-CSF or DNA and subsequently boosted with antigenized antibody γ1NANP in IFA. Inoculation of DNA/GM-CSF but not DNA induced IgGl antibodies during the primary response. A booster injection with antibody γ1NANP in IFA increased the IgGl titer in DNA/GM-CSF primed mice. The antibody titer was on average 4 fold higher (4.1-4.4 vs 3.5-3.8) in mice primed with DNA/GM-CSF than in mice primed with DNA alone (Table 6).

**Table 6. 1g Gl responses in mice primed with DNA/GM-CSF and boosted with antigenized antibody protein.**

| Experiment No. ^{a)} | Well coating | **Primary response** Immunogen | | | **Secondary response** Immunogen | | |
|---|---|---|---|---|---|---|---|
| | | DNA | DNA/GM-CSF | Enhancement (fold) | DNA | DNA/GM-CSF | Enhancement (fold) |
| 1 | NANPn | <200(2.3) ^{b)} | 1.600(3.2) | 8 | 3.200(3.5) | 12.800(9.1) | 4 |
| 2 | | <200(2.3) | 1.600(3.2) | 8 | 6.400(3.8) | 25.600(9.4) | 4 |
| 1 | γNANP | <200(2.3) | 12.800(4.1) | 64 | 102.400(5.0) | 409.600(5.6) | 4 |
| 2 | | <200(2.3) | 6.400(3.8) | 32 | 51.200(4.7) | 204.800(5.3) | 4 |

| | | | | | | | |
|---|---|---|---|---|---|---|---|
| a) The two experiments represented were run independently. Each group consisted of four mice. Priming was performed by a single intrasplenic inoculation of DNA or DNA/GM-CSF. The booster immunization was given at day 35 with γ1NANP antibody in IFA. Pooled sera were tested against the synthetic peptide (NANP)n or the whole antigenized antibody as indicated. b) Values refer to antibody titers expressed as reciprocal of the last positive dilution. In parentheses are indicated the corresponding log 10 titers. | | | | | | | |

GM-CSF heightens the anamnestic response induced by injection of *P*. *falciparum* sporozoites. Mice primed by inoculation of plasmid DNA respond to a booster immunization by *P*. *falciparum* sporozoites with a typical secondary response (see Example III). Booster by parasites yielded 4 fold higher IgGl anti-NANP antibody titers in mice primed with DNA/GM-CSF as compared with mice primed with DNA only (Log 4.7 vs. 4.1) (Figure 7, left panel). No antibodies were detected in mice primed with saline and boosted with sporozoites (negative controls). The effect on IgM antibodies was minimal (Figure 7, right panel). Therefore, GM-CSF given during priming heightens the IgGl memory response irrespective of the composition of the antigen used in the booster immunization.

### EXAMPLE VI

### Activation of CD4 T Cells by Somatic Transgenesis Induces Generalized Immunity of Uncommitted T Cells and Immunologic Memory

This example describes the activation of CD4 T cells with administration of a nucleic acid molecule encoding an epitope.

The protocols used are described below (Gerloni et al., J. Immunol. 162:3782-3789 (1999)).

Plasmids γ1NV²NA³ was engineered as described in Example IV. Plasmid γ1NANP is described in Figure 1. Recombinant antigenized antibodies γ1NV²NA³ and γ1NANP were produced in transfectoma cells and purified as described in Example IV (Sollazzo et al., *supra,* 1990a).

Mice were inoculated intraspleen with 100 µg of plasmid DNA in 50 µl of sterile saline solution as previously described in Example I. Booster injections were administered on day 90, 110, 120 and 150 after priming by a single subcutaneous injection (50 µg per mouse) of affinity-purified γ1NV²NA³ antibody emulsified in incomplete Freunds' adjuvant (IFA).

At the time of harvest, mice were sacrificed and the lymph nodes and spleens removed. Single cell suspensions were cultured (10⁶ cells/ml) in RPMI 1640 medium (Irvine Scientific; Santa Ana CA) supplemented with Hepes buffer, glutamine, 7.5% fetal calf serum and 50 µM 2-mercaptoethanol, in the presence or absence of synthetic peptides NANPNVDPNANP or NANPNANPNANP (50 µg/ml) in triplicate. The cells were incubated at 37°C in 10% CO₂ for 3 days. (³H)-Thymidine was added at 1µCi/well and the cells were incubated for 16-18 hours at 37°C. Cells were harvested onto glass fiber filter mats using a Tomtec cell harvester and the radioactivity was measured in a liquid scintillation counter (Betaplate; Wallac; Tuku Finland). Results are expressed as Stimulation Index (S.I.) calculated as the ratio of (counts per minute of cells cultured in the presence of synthetic peptide)/(counts per minute of cells cultured in the absence of peptide). Concanavalin A (ConA) stimulation was used as a polyclonal activator and positive control.

CD4⁺ and CD8⁺ T cells were isolated by antibody plus complement-mediated depletion from splenocytes of mice immunized 7 days earlier by DNA inoculation. Briefly, cell suspensions (30x10⁶ cells/ml) were treated with monoclonal antibody to CD8 (3.155) or CD4 (RL172) for 30 minutes on ice. After washing, anti-T cell antibodies were cross-linked with a mouse anti-rat (MAR 18.5) monoclonal antibody for 30 minutes on ice and rabbit complement was added twice for 30 minutes at 37°C. The cell suspension was then washed twice and resuspended at the concentration of 5x10⁶ cells/ml in RPMI (Irvine Scientific). The purity of the separated cell fractions was assessed by analysis on a FACScan with Cellquest software (Becton & Dickinson, Mountain View, CA) using phycoerythrin (PE)-conjugated anti-CD4 and fluorescein isothiocyanate (FITC)-conjugated anti-CD8 monoclonal antibodies (Pharmingen, San Diego CA).

Culture supernatants were harvested 40 hours after initial seeding and were stored at -20°C. The supernatants from three separate triplicate cultures were pooled for each mouse. IL-2 activity was determined in a bioassay utilizing the IL-2- and IL-4-dependent NK.3 cells in the presence of anti-IL4 (purified from the 11B11 cell line, ATCC) . Briefly, 100 µl (1:2 dilution in medium) of 40 hour culture supernatants were added in duplicate to 100 µl of NK.3 cells (10⁶/ml) and incubated for 36 hours. (³H)-Thymidine was added at 1 µCi/well during the last 12 hours. Cells were harvested as specified above. Results are expressed as counts per minute.

IL-4, IL-5 and IFN-γ were measured in the same 40 hours culture supernatants by ELISA as described previously using the antibodies 11811 and biotinylated anti-IL-4 (BVD6, Pharmigen), TRFK5 and biotinylated TRFK9 and R46A-2 and biotin-XMG1.2 (Pharmingen), respectively. Standard curves were constructed with purified IL-2, IL-4, IL-5 and IFN-g (supernatants from the respective X63.Ag. cell lines). Tests were done in duplicate.

As a source of antigen presenting cells (APC), spleen cells from unprimed mice were used and cultured with LPS/Dextran (25 µg/ml) for 24 hours and treated for 30 min at 37°C with 25 µg/ml mitomycin C (Sigma). Before use, spleen cells from naive, primed, or primed and boosted mice were mixed with 2x10⁶/ml APC in 96-well flat-bottom plates in the presence of 50 µg/ml synthetic peptide NANPNVDPNANP(-NVDP-). Each dilution of cells was plated in replicates of 48. Supernatants were harvested after 36 hours and 20 µl from each culture was tested for IL-2 activity using the NK.3 cell line. Single cultures supernatants were considered positive when the value of ³H-thymidine incorporation was greater than the mean of the replicate control cultures with no antigen plus two standard deviations. Frequencies of cytokine producing cells were calculated using the program described by Waldman and were calculated using maximum likelihood analysis.

Spleen cells harvested 7 days after a single intraspleen inoculation of 100 µg of γ1NV²NA³ DNA proliferated in culture after re-stimulation with the antigenized antibody expressing the Th cell determinant or the corresponding 12mer Th cell determinant peptide (Figure 8A). Proliferation occurred when cells were cultured with the T- (-NVDP-) but not the B- [(NANP)3] cell peptide demonstrating specific activation by the heterologous peptide in CDR2. Proliferation after culture with the antigenized antibody expressing -NVDP-also suggests that the CDR2 peptide within the antibody molecule is processed and presented by APC. When compared with the proliferative response of cells from mice immunized with the antigenized antibody in CFA, STI induced a response of similar or greater magnitude. Specific activation of T cells was accompanied by marked production of IL-2 (Figure 8B). The lower amounts of IL-2 measured in cultures re-stimulated *in vitro* with the -NVDP- peptide most likely reflect a higher consumption as cells in these cultures were proliferating to a greater extent.

Splenocytes harvested on day 7 and 14 were also assayed for production of IFN-γ, IL-4 and IL-5 to assess whether any polarization to Type 1 and Type 2 phenotype had occurred (Figure 9). Both IFN-γ and IL-4 were detected, albeit in different amounts and IL-5 was absent. Since IFN-γ specific activity is on average 100 fold lower than IL-4, and IL-4 is typically secreted in much lower quantities than IFN-γ, these results indicate that both cytokines are produced proportionally and that cells activated through STI remain, by and large, uncommitted (Th0).

Activated cells were determined to be CD4⁺ T lymphocytes. CD4⁺ T cells were formally identified as the cell population proliferating and making cytokines. Spleen cells from mice immunized 7 days earlier were depleted of CD4⁺ and CD8⁺ cells by treatment *in vitro* with monoclonal antibodies specific for CD8 or CD4 plus complement. By flow-cytometry the purity of the two populations was 94 % (CD4) and 99 % (CD8), respectively (Figure 10C and 10D). The two cell populations were then cultured *in vitro* with the addition of fresh APC from naive mice and synthetic peptide -NVDP-. Proliferation occurred in the CD4⁺ but not in the CD8⁺ T cell population (Figure 10E). Similarly, IL-2 production was detected only in the CD4⁺ T cell population (Figure 10F). These results demonstrate that STI selectively activates CD4⁺ T lymphocytes.

T cell immunity was found to spread to other secondary lymphoid organs. Germane to the present studies was to determine the extent to which priming induces generalized T cell activation. In a first set of experiments, spreading of immunity to other secondary lymphoid organs was monitored by measuring cell proliferation and IL-2 production in a pool of inguinal, mesenteric and cervical lymph node cells. Seven days after DNA inoculation cells of the lymph node pool proliferated specifically upon re-stimulation *in vitro* with the -NVDP- but not with the B-cell epitope peptide (Figure 11A). When compared with spleen cells, proliferation in lymph nodes was of a lesser magnitude. On day 14, the magnitude of the response in lymph node cells increased markedly reaching values comparable to spleen cells. On day 21, only residual proliferative activity existed in both lymph node and spleen cells. The magnitude and specificity of the proliferative responses were reflected by the levels of IL-2 in the corresponding culture supernatants (Figure 11B). These kinetic analyses revealed that T cell activation in lymph nodes parallels that in the origan in which the process of immunity was initiated. Cells of lymph nodes collected according to precise anatomical distribution, lower (popliteal, caudal, sciatic and lumber), middle (mesenteric, renal and epigastric) and upper (axillary, brachial, deep and superficial cervical) had similar T cell proliferation and IL-2 production (Figure 11D and 11E).

Analysis of the tempo of these responses in relation to other parameters of STI revealed something interesting. When the ratio between the stimulation indexes in lymph nodes and spleen was calculated, it became evident that, by day 14, T cell responsiveness in lymph nodes was prevalent. Moreover, the peak of the proliferative response in lymph nodes appeared to correlate with the peak values of transgenic Ig in the serum (Figure 11C). The results indicate that a pattern of proportionality exists between secretion of transgenic Ig and spreading of T cell immunity.

The effects of linked recognition of Th and B cell epitopes on the antibody response was determined. Mice given the transgene coding for both the Th cell determinant and the B-cell epitope produced consistently higher antibody titers than mice immunized with the B-cell epitope-containing gene (Figure 12). Second, specific activation of Th cells by the NVDP- determinant was determined to be sufficient to promote the IgM to IgGl switch. Mice given the Th/B double-epitope transgene developed IgM and IgGl antibodies (Figure 12). These results indicate that T cell immunity triggered by the Th cell determinant in linked association with a B-cell epitope optimizes the B-cell response by heightening the antibody titer and by promoting isotype switch.

The response to secondary exposure to antigen *in vivo* was determined. The frequency of antigen-responsive T cells was much higher after booster immunization with antigenized antibody γ1NV²NA³ (50 µg) in incomplete Freunds' adjuvant (IFA) (Table 7). For comparative purposes, LDA studies were also performed 4 and 7 days after single DNA inoculation (Table 7). On day 4 and 7 the frequency was 1/90,200 (group II) and 1/50,500 (group III), respectively. Four days after priming with protein antigen in IFA, the frequency was 1/60,000 (group VII). The average frequency during the memory response was 1/21,900 that is 2.5-4 times higher. Table 7 also shows that early after DNA priming
antigen-responsive T cells were enriched 75 fold over naive precursors but dropped to 1/424,500 (group V) by day 110. Collectively, these results indicate that priming by STI establishes T cell memory. Re-encounter with antigen induced a faster and higher specific response.

**Table 7. Frequency of CD4 T cells specific for the Th determinant.**

| Group | Priming | Days After Priming | Booster | Day of Booster | Frequency of CD4 cells^{a} |
|---|---|---|---|---|---|
| I | None | | | | 1/1, 558, 000^{a} |
| II | DNA | 4 | | | 1/90, 200 |
| III | DNA | 7 | | | 1/50, 500^{a} |
| IV | DNA | 14 | | | 1/36, 400 |
| V | DNA | 110 | | | 1/424, 500^{b} |
| VI | DNA | | Protein | 110 | 1/21, 900^{b,c} |
| VII | None | | Protein | | 1/60, 000^{b} |

| | | | | | |
|---|---|---|---|---|---|
| ^{a} Values represent the average of two independent experiments. ^{b} Values represent the average of three independent experiments. The booster immunization was performed on day 90-110. ^{c} Spleen cells were harvested and put in culture 4 days after booster immunization. | | | | | |

The results disclosed herein indicate that STI is an effective way to activate CD4 T cells and establish durable T cell memory. The frequency of antigen-reactive T cells increased 3-4 fold in a long term primed animal and again several fold after booster immunization. In addition, the response was faster than the primary response, consistent with a functional definition of immunologic memory. In all likelihood, early effector T cells gave rise to resting memory cells, which are known to re-circulate as a pool through spleen and lymph nodes until they are sequestered again by antigen 24-48 hours later.

### EXAMPLE VII

### Somatic Transgene Immunization Activates CD8 T Cells and Protects Against Virus Challenge

This example describes the activation of CD8 T cells with administration of a nucleic acid molecule encoding an epitope from the influenza virus A/PR8.

The protocols used are in part described below (Billetta et al., Eur. J. Immunol. 25:776-783 (1995)).

A H-chain gene was engineered to express in the third complementarity-determining region (CDRs3) 13 amino acid residues from the sequence of the A/PR/8/34 influenza virus nucleoprotein (NP) antigen (Figure 13). This NP peptide is presented in association with the Db allele in H-2b mide.

Mice were inoculated with 100 µg of plasmid DNA per inoculation. All DNA inoculations were done intraspleen as indicated under Example I. Groups of mice were additionally boosted after 12 weeks with 50 µg of synthetic peptide ASNENNETMESSTL (amino acid residues 366-374) (NP peptide) emulsified in incomplete Freunds' adjuvant. Control groups consisted in mice immunized twice with 50 µg of NP peptide emulsified in concomplete Freunds' adjuvant (positive control) or mice of the same age group that did not receive any treatment (negative control).

Mice were challenged intranasally with 10xLD50 dose of infectious homologous virus. After challenge mice were monitored for loss of weight and survival.

Cytotoxicity was tested on spleen cells using a 4 hour 51Cr release assay. Briefly, RMAS (H2b) target cells were labeled with Na51CrO4 (150 mCi/l x 106 cells) for 1 hour at 37°C in an atmosphere of 5% CO2 with or without NP peptide (10µg/ml), then washed and resuspended in culture medium supplemented with 10% FCS. One hundred µl of 51Cr-labeled target cells (2.5 x 105 cells/ml) were mixed with effector cells in 100 µl at various (100:1) effector:target (E:T) ratio. The plates were incubated for 4 hours at 37°C in 5% CO2, then centrifuged at 500 g for 4 minutes. One hundred µl of supernatant were removed and counted in a gamma counter. Spontaneous and maximal 51Cr releases were determined by incubating target cells in medium alone or in the presence of 1% Triton 100x, respectively. Percent cytotoxicity was calculated from triplicate wells as follows: [experimental release - spontaneous release / maximal release - spontaneous release] x 100.

Early studies *in vitro* demonstrated that a B cell harboring an Ig H chain transgene process and present in a T cell peptide to cytotoxic (CD8) T cells, and are lysed with high efficiency (Billetta et al., Eur. J. Immunol. 25:776-783 (1995)). For instance, B-lymphoma cells (Db) transfected with the H chain gene engineered to express in the third CDR the NP peptide ASNENNETMESSTL were efficiently killed by specific CTL in a dose-dependent manner indicating intracellular processing and presentation of the NP peptide at the surface of the cell.

In a series of experiments, it was shown that C57BL6 mice inoculated with this transgene develop a CTL response. Spleen cells from inoculated mice were harvested three weeks after immunization and tested for their ability to kill NP peptide-pulsed RMAS target cells in a conventional cytotoxicity assay. RMAS cells without peptide served as a control. In this assay we found that between 60-75% of mice had generated a cytotoxic T cell response specific for the influenza NP peptide.

Protection and induction of memory CTL was also documented (see Figure 14). In the experiment shown, mice (10 per group) were vaccinated wither via STI or with synthetic peptide in incomplete Freunds' adjuvant. A group of mice remained untreated and served as control. Three months after vaccination mice received an intranasal challenge with 10xLK50 dose of infectious influenza virus (i.e. 10 times the lethal dose of r50% of mice). As shown, all untreated mice vaccinated with synthetic peptide in adjuvant died by day 11. As shown, the majority (50 and 60%) of mice vaccinated by somatic transgene immunization survived.

### EXAMPLE VIII

### Positive Reciprocal Regulation Between Two Th Cell Epitope During Somatic Transgene Immunization

This example describes the activation *in vivo* of CD4 T cells against determinants of a tumor antigen per se unable to induce a cellular response. This is obtained by immunization with nucleic acid molecule encoding tumor epitopes in linked association with a dominant T cell epitope of the malaria parasite.

Two H-chain genes were engineered to express in the CDR3 two amino acid sequences (VTSAPDTRPAP and DTRP3) from the tandem repeat of the tumor antigen MUC-1 (Gendler et al., Proc Natl Adad Sci USA, 84:6060-6064 (1987)). Each gene coding for a single epitope of the MUC-1 antigen was also engineered to code in the CDR2 for the Th cell determinant NANPNVDPNANP from the outer coat of the malaria parasite *P*. *Falciparum* (Nardin et al., Science 246:1603-1606 (1989)). The corresponding plasmid vector is termed γ1NV2VTSA3 (Figure 15) and γ1NV2DTRP3.

Plasmid DNA coding for just the MUC-1-derived peptide sequence were unable to induce a proliferative response *in vivo.* However, plasmids γ1NV2VTSA3 and γ1NV2DTRP3 induced a strong response against the respective MUC-1 epitope (Figure 16). None of the eight mice immunized with DNA coding for the single MUC-1 epitope alone developed a T cell response. In converse a response occurred in all mice immunized with a gene coding in linked association for the MUC-1 epitope and the heterologous Th cell determinant from the malaria parasite.

These results indicate that weak immunogenic epitopes can be rendered immunogenic by association with a strong heterologous Th-cell determinant. This finding is relevant for the development of a MUC-1-based vaccine but also for the development of T cell immunity against other tumor antigens.

These results indicate that a linked association of two Th cell determinants T cells can be exploited to immunize against weak T cell determinants, for instance of tumor antigens. These results indicate that a linked Th/Th association in a gene that is used for immunization along the principles of somatic transgene immunization can render immunogenic an otherwise poorly or non-immunogenic Th cell determinant. These results indicate that this principle is applicable to vaccines against all antigens against which strong T cell immunity is desired.

### EXAMPLE IX

### Ex Vivo Somatic Transgene Immunization Induces T cell Immunity

This example describes the induction of antigen specific CD4 T cells using *ex vivo* STI. In a first *in vitro* step, normal spleen lymphocytes were transfected with plasmid γ1NV²NA³. Twenty-four hours after transfection the lymphocytes were injected intravenously into normal mice.

In the experiment shown (Table 8) mice were injected with different numbers of transfected lymphocytes in 200 ml of sterile saline i.v. in the vein of the tail. Mice were sacrificed 14 days days after injection of transfected cells. Single spleen cell suspensions were cultured (10⁶ cells/ml) in RPMI 1640 medium (Irvine Scientific; Santa Ana, CA) supplemented with Hepes buffer, glutamine, 7.5% fetal calf serum and 50 µM 2-mercaptoethanol, in the presence or absence of synthetic peptides NANPNVDPNANP or NANPNANPNANP (50 µg/ml) in triplicate. The cells were incubated at 37°C in 10% CO₂ for 3 days. (³H)-Thymidine was added at 1µCi/well and the cells were incubated for 16-18 hours at 37°C. Cells were harvested onto glass fiber filter mats using a Tomtec cell harvester and the radioactivity was measured in a liquid scintillation counter (Betaplate; Wallac; Tuku Finland). Results are expressed as Stimulation Index (S.I.) calculated as the ratio of (counts per minute of cells cultured in the presence of synthetic peptide)/(counts per minute of cells cultured in the absence of peptide). Concanavalin A (ConA) stimulation was used as a polyclonal activator and positive control. Sera were used for detection of transgenic product (TgIg) and the presence of antibodies against TgIg.

The results described in Table 8 shows that a specific proliferative response was detected in all mice over a range of 20,000 to 70 positive cells injected/mouse. The proliferative response followed a dose-response curve, and the response was specific. Control mice injected with transgenic lymphocytes harboring the transgene lacking the Th cell determinant failed to respond at any of the cell concentration tested.

**Table 8. Ex vivo STI induces a CD4 T cell response. A dose-response analysis.**

| Group | No. of Cells injected | Cells Transfected with | |
|---|---|---|---|
| | | γ1NV²NA³ | γ1NA³ |
| I | 20,000 | 42,125 | 2,946 |
| | | 28,113 | 255 |
| II | 5,000 | 26,108 | 109 |
| | | 28,133 | 866 |
| III | 1,250 | 11,597 | 849 |
| | | 28,464 | 242 |
| IV | 300 | 11,381 | 199 |
| | | 8,110 | 238 |
| V | 70 | 4,070 | 718 |
| | | 13,255 | 477 |

Naive C57B1/6 mice were injected i.v. with syngeneic lymphocytes transfected with plasmid γ1NV²NA³. Groups of two mice each received a single injection of cells (20,000 to 70 cells/mouse) harboring the transgene. Two weeks after cell immunization, mice were sacrificed and the spleen cells prepared and tested in a conventional CD4 T cell proliferation assay in the presence of the -NVDP- peptide or the (NANP)₃ peptide as a control. Control mice were similarly immunized with an equal number of spleen cells harboring a control transgene, plasmid γ1NA³, coding for the (NANP)₃ peptide but not for the CD4 T cell determinant -NVDP. Results are expressed as cpm of cultures re-stimulated *in vitro* with the -NVDP- peptide minus cpm of cultures with medium alone. Values (cpm) of control cultures re-stimulated with the B cell epitope (NANP)₃ are not shown because equal to values (cpm) of cultures with medium alone.

The results disclosed herein indicate that *ex vivo* STI is an effective way to activate CD4 T cells. Antigen specific immunity was readily induced by intravenous injection of normal lymphocytes transfected with an Ig H chain gene coding in one CDR for a Th cell determinant. Immunization via *ex vivo* STI induced a proliferative response with the characteristic of a dose-response immunization.

### EXAMPLE X

### Somatic Transgenesis Functions in vitro for Human B Cells

This example describes the spontaneous transfection of human B cells using bacterial plasmid DNA coding for an immunoglobulin gene.

Raji (MHC class II⁺) and RJ2.2.5 (a MHC class II⁻ variant) were cultured in RPMI-1640 containing 10%FCS supplemented with 2% glutamine. Plasmid DNA γ1NANP and PCR methodologies are as described in Example II.

Raji (MHC Class II⁺) and RJ2.2.5 (a MHC class II⁻ variant) were harvested and washed throughly with sterile saline, counted and redistributed at various concentrations in 300 µl of phosphate buffered saline. 5 µg of plasmid DNA (γ1NANP) was added to the cell suspension and incubated at 37 °C, for 1 hour in a 5% CO₂ atmosphere. After the incubation the cells were washed with saline and put in complete culture medium and grown at 37 °C, 5% CO₂ for 24 hours. Uptake and transfection were assessed on cells harvested 24 hours later. Genomic DNA was extracted using the QIAamp Blood Kit (Qiagen) and subjected to two-rounds of nested PCR using VDJ specific primers (see Example II). The PCR products were analyzed on a 1% agarose gel with ethidium bromide stain. After 24 hours the transgene was detected with PCR in both the Raji and RJ2.2.5 cells, suggesting uptake and integration of the transgene. In a different experiment the total RNA of 10⁵ transfected cells was extracted in a single-step after 7 days of culture using guanidinium thicyanate phenol-chloroform. A murine transfectoma cell line was used as a positive control. By RT-PCR, RNA coding for the H chain transgene product was detected in transfected Raji but not in untransfected Raji cells.

## Claims

1. Use of B lymphocytes obtained from an individual or from an immunologically compatible individual to which nucleic acid molecules have been administered *ex vivo,* wherein the nucleic acid molecules comprise a hematopoietic cell-specific expression element operationally linked to a nucleic acid sequence encoding one or more heterologous epitopes, for the preparation of a pharmaceutical composition for administration to the individual for stimulating an immune response.

2. The use of claim 1, wherein said B lymphocyte is derived from blood or a lymphoid tissue selected from the group consisting of spleen, lymph nodes, mucosa-associated lymphoid tissue (MALT), tonsils, Peyer's patches, nasal-associated lymphoid tissue (NALT), Waldeyer's ring, and urogenital lymphoid tissue.

3. The use of claim 1 or 2, wherein said expression element functions in B cell.

4. The use of claims 1 to 3, wherein said epitope stimulates an antibody response.

5. The use of claims 1 to 3, wherein said epitope stimulates a CD4 T cell response.

6. The use of claims 1 to 3, wherein said epitope stimulates a CD8 T cell response.

7. The use of claims 1 to 3, wherein said epitope stimulates a CD4 T cell response and a CD8 T cell response.

8. The use of claims 1 to 3, wherein one of said epitopes stimulates an antibody response and one or more second epitopes stimulates a CD4 T cell response and a CD8 T cell response.

9. The use of claims 1 to 8, wherein said epitope is expressed as a fusion with a cytokine.

10. The use of claim 9, wherein said cytokine is selected from the group consisting of granulocyte-macrophage colony-stimulating factor, interleukin-2, interleukin-4, interferon-γ, interleukin-5, interleukin-6, interleukin-10 and interleukin-12.

11. The use of claims 1 to 8, wherein said nucleic acid molecule encodes an immunoglobulin molecule containing said heterologous epitope, wherein said epitope is inserted within a complementarity-determining region (CDR) of said immunoglobulin molecule.

12. The use of claim 11, wherein said immunoglobulin comprises a variable region.

13. The use of claim 12, wherein said variable region is a heavy chain variable region.

14. The use of claim 12, wherein said variable region is a light chain variable region.

15. The use of claim 11, wherein said immunoglobulin molecule comprises a heavy chain.

16. The use of claim 11, wherein said immunoglobulin molecule comprises a light chain.

17. A nucleic acid molecule comprising a hematopoietic cell-specific expression element which functions in a B cell operationally linked to a nucleic acid sequence encoding a heterologous polypeptide, wherein said heterologous polypeptide comprises two or more T cell epitopes, wherein said T cell epitopes are selected from the group consisting of a CD4 and a CD8 epitope, two CD4 epitopes, and two CD8 epitopes.

18. The nucleic acid of claim 17, wherein said heterologous polypeptide further comprises one or more B cell epitopes.

19. The nucleic acid molecule of claim 17, wherein said nucleic acid sequence encodes a polypeptide expressed as a fusion with a cytokine.

20. The nucleic acid molecule of claim 19, wherein said cytokine is selected from the group consisting of granulocyte-macrophage colony-stimulating factor, interleukin-2, interleukin-4, interferon-γ, interleukin-5, interleukin-6, interleukin-10 and interleukin-12.

21. The nucleic acid molecule of claims 17 to 20, wherein said nucleic acid sequence encodes an immunoglobulin molecule containing said two or more T cell epitopes and wherein said two or more T cell epitopes are inserted within a complementarity-determining region (CDR) of said immunoglobulin molecule.

22. The nucleic acid molecule of claim 21, wherein said immunoglobulin comprises a variable region.

23. The nucleic acid molecule of claim 22, wherein said variable region is a heavy chain variable region.

24. The nucleic acid molecule of claim 22, wherein said variable region is a light chain variable region.

25. The nucleic acid molecule of claim 21, wherein said two or more epitopes are inserted in two CDRs.

26. The use of claim 1, wherein the nucleic acid molecule is a non-viral vector and the hematopoietic cell-specific expression element is a B cell-specific expression element, which is operationally linked to a nucleic acid sequence encoding a heterologous polypeptide, wherein said nucleic acid molecule expresses said heterologous polypeptide when targeted to B cells.

27. The use of claim 1, wherein the nucleic acid encodes two or more heterologous T cell epitopes.

## Patentansprüche

1. Verwendung von B-Lymphozyten, welche von einem Individuum oder von einem immunologisch kompatiblen Individuum erhalten wurden, dem *ex vivo* Nukleinsäuremoleküle verabreicht wurden, wobei die Nukleinsäuremoleküle ein für hämatopoietische Zellen spezifisches Expressionselement funktionell verbunden mit einer für ein oder mehrere heterologe Epitope kodierenden Nukleinsäuresequenz umfassen, zur Herstellung einer pharmazeutischen Zusammensetzung zur Verabreichung an das Individuum zur Stimulation einer Immunantwort.

2. Die Verwendung nach Anspruch 1, **dadurch gekennzeichnet, dass** der B-Lymphozyt aus Blut oder einem lymphoiden Gewebe aus der Gruppe bestehend aus Milz, Lymphknoten, Mucosaassoziiertem lymphoiden Gewebe (mucosa-associated lymphoid tissue, MALT), Mandeln, Peyer's Patches, Nasen- assoziiertem lymphoiden Gewebe (nasal-associated lymphoid tissue, NALT), Waldeyer's Ring und urogenitalem lymphoiden Gewebe ausgewählt ist.

3. Die Verwendung nach Anspruch 1 oder 2, **dadurch gekennzeichnet, dass** das Expressionselement in B-Zellen funktioniert.

4. Die Verwendung nach Ansprüchen 1 bis 3, **dadurch gekennzeichnet, dass** das Epitop einer Antikörperantwort stimuliert.

5. Die Verwendung nach Ansprüchen 1 bis 3, **dadurch gekennzeichnet, dass** das Epitop eine CD4 T-Zell-Antwort stimuliert.

6. Die Verwendung nach Ansprüchen 1 bis 3, **dadurch gekennzeichnet, dass** das Epitop eine CD8 T-Zell-Antwort stimuliert.

7. Die Verwendung nach Ansprüchen 1 bis 3, **dadurch gekennzeichnet, dass** das Epitop eine CD4 T-Zell-Antwort und eine CD8 T-Zell-Antwort stimuliert.

8. Die Verwendung nach Ansprüchen 1 bis 3, **dadurch gekennzeichnet, dass** eins der Epitope eine Antikörperantwort stimuliert und ein oder mehrere zweite Epitope eine CD4 T-Zell-Antwort und eine CD8 T-Zell-Antwort stimulieren.

9. Die Verwendung nach Ansprüchen 1 bis 8, **dadurch gekennzeichnet, dass** das Epitop in Fusion mit einem Zytokin exprimiert ist.

10. Die Verwendung nach Anspruch 9, **dadurch gekennzeichnet, dass** das Zytokin ausgewählt ist aus der Gruppe bestehend aus Granulozyten-Makrophagen Kolonie-stimulierender Faktor, Interleukin-2, Interleukin-4, Interferon-γ, Interleukin-5, Interleukin-6, Interleukin-10 und Interleukin-12.

11. Die Verwendung nach Ansprüchen 1 bis 8, **dadurch gekennzeichnet, dass** das Nukleinsäuremolekül für ein Immunglobulinmolekül kodiert, welches das heterologe Epitop enthält, wobei das Epitop in eine komplementaritätsbestimmende Region (CDR) des Immunglobulinmoleküls eingefügt ist.

12. Die Verwendung nach Anspruch 11, **dadurch gekennzeichnet, dass** das Immunglobulin eine variable Region umfasst.

13. Die Verwendung nach Anspruch 12, **dadurch gekennzeichnet, dass** die variable Region eine variable Region der schweren Kette ist.

14. Die Verwendung nach Anspruch 12, **dadurch gekennzeichnet, dass** die variable Region eine variable Region der leichten Kette ist.

15. Die Verwendung nach Anspruch 11, **dadurch gekennzeichnet, dass** das Immunglobulinmolekül eine schwere Kette umfasst.

16. Die Verwendung nach Anspruch 11, **dadurch gekennzeichnet, dass** das Immunglobulinmolekül eine leichte Kette umfasst.

17. Ein Nukleinsäuremolekül, umfassend ein für hämatopoietische Zellen spezifisches Expressionselement, welches in einer B-Zelle funktioniert, funktionell verbunden mit einer für ein heterologes Polypeptid kodierenden Nukleinsäuresequenz, wobei das heterologe Polypeptid zwei oder mehr T-Zell-Epitope umfasst, wobei die T-Zell-Epitope ausgewählt sind aus der Gruppe bestehend aus einem CD4 und einem CD8 Epitop, zwei CD4 Epitopen und zwei CD8 Epitopen.

18. Die Nukleinsäure nach Anspruch 17, **dadurch gekennzeichnet, dass** das heterologe Polypeptid ferner ein oder mehr B-Zell-Epitope umfasst.

19. Das Nukleinsäuremolekül nach Anspruch 17, **dadurch gekennzeichnet, dass** die Nukleinsäuresequenz für ein Polypeptid kodiert, welches in Fusion mit einem Zytokin exprimiert ist.

20. Das Nukleinsäuremolekül nach Anspruch 19, **dadurch gekennzeichnet, dass** das Zytokin ausgewählt ist aus der Gruppe bestehend aus Granulozyten-Makrophagen Kolonie-stimulierender Faktor, Interleukin-2, Interleukin-4, Interferon-γ, Interleukin-5, Interleukin-6, Interleukin-10 und Interleukin-12.

21. Das Nukleinsäuremolekül nach Ansprüchen 17 bis 20, **dadurch gekennzeichnet, dass** die Nukleinsäuresequenz für ein Immunglobulinmolekül kodiert, welches zwei oder mehr T-Zell-Epitope enthält und wobei die zwei oder mehr T-Zell-Epitope in einer komplementaritätsbestimmenden Region (CDR) des Immunglobulinmoleküls eingefügt sind.

22. Das Nukleinsäuremolekül nach Anspruch 21, **dadurch gekennzeichnet, dass** das Immunglobulin eine variable Region umfasst.

23. Das Nukleinsäuremolekül nach Anspruch 22, **dadurch gekennzeichnet, dass** die variable Region eine variable Region der schweren Kette ist.

24. Das Nukleinsäuremolekül nach Anspruch 22, **dadurch gekennzeichnet, dass** die variable Region eine variable Region der leichten Kette ist.

25. Das Nukleinsäuremolekül nach Anspruch 21, **dadurch gekennzeichnet, dass** die zwei oder die mehreren Epitope in zwei CDRs eingefügt sind.

26. Die Verwendung nach Anspruch 1, **dadurch gekennzeichnet, dass** die Nukleinsäure ein nicht-viraler Vektor ist und das für hämatopoietische Zellen spezifische Expressionselement ein für B-Zellen spezifisches Expressionselement ist, welches funktionell mit einer für ein heterologes Polypeptid kodierenden Nukleinsäuresequenz verbunden ist, wobei das Nukleinsäuremolekül das heterologe Polypeptid exprimiert, wenn es auf B-Zellen gerichtet ist.

27. Die Verwendung nach Anspruch 1, **dadurch gekennzeichnet, dass** die Nukleinsäure für zwei oder mehr heterologe T-Zell-Epitope kodiert.

## Revendications

1. Utilisation de lymphocytes B, prélevés chez un individu ou chez un individu compatible du point de vue immunologique, dans lesquels ont été introduites *ex vivo* des molécules d'acide nucléique qui comprennent un élément d'expression, spécifique pour cellules hématopoïétiques et raccordé de manière opérationnelle à une séquence d'acide nucléique qui code un ou plusieurs épitopes hétérologues, en vue de la préparation d'une composition pharmaceutique conçue pour être administrée à l'individu afin de stimuler une réponse immune.

2. Utilisation conforme à la revendication 1, pour laquelle lesdits lymphocytes B proviennent du sang ou d'un tissu lymphoïde choisi dans l'ensemble formé par les suivants : rate, ganglions lymphatiques, tissus lymphoïdes associés aux muqueuses (tissus MALT), amygdales, plaques de Peyer, tissus lymphoïdes associés à la cavité nasale (tissus NALT), anneau de Waldeyer, et tissu lymphoïde urogénital.

3. Utilisation conforme à la revendication 1 ou 2, dans laquelle ledit élément d'expression fonctionne dans les lymphocytes B.

4. Utilisation conforme à l'une des revendications 1 à 3, dans laquelle ledit épitope stimule une réponse par anticorps.

5. Utilisation conforme à l'une des revendications 1 à 3, dans laquelle ledit épitope stimule une réponse par lymphocytes T à CD4.

6. Utilisation conforme à l'une des revendications 1 à 3, dans laquelle ledit épitope stimule une réponse par lymphocytes T à CD8.

7. Utilisation conforme à l'une des revendications 1 à 3, dans laquelle ledit épitope stimule une réponse par lymphocytes T à CD4 et une réponse par lymphocytes T à CD8.

8. Utilisation conforme à l'une des revendications 1 à 3, dans laquelle l'un desdits épitopes stimule une réponse par anticorps, et un ou plusieurs autre(s) épitope(s) stimule(nt) une réponse par lymphocytes T à CD4 et une réponse par lymphocytes T à CD8.

9. Utilisation conforme à l'une des revendications 1 à 8, dans laquelle ledit épitope est exprimé sous la forme de produit de fusion avec une cytokine.

10. Utilisation conforme à la revendication 9, dans laquelle ladite cytokine est choisie dans l'ensemble formé par les suivantes : facteur GM-CSF (facteur de stimulation des colonies de granulocytes et macrophages), interleukine-2, interleukine-4, interféron gamma, interleukine-5, interleukine-6, interleukine-10 et interleukine-12.

11. Utilisation conforme à l'une des revendications 1 à 8, dans laquelle ladite molécule d'acide nucléique code une molécule d'immunoglobuline qui comporte ledit épitope hétérologue, lequel épitope est inséré au sein d'une région CDR (région déterminant la complémentarité) de ladite molécule d'immunoglobuline.

12. Utilisation conforme à la revendication 11, dans laquelle ladite immunoglobuline comporte une région variable.

13. Utilisation conforme à la revendication 12, dans laquelle ladite région variable est une région variable de chaîne lourde.

14. Utilisation conforme à la revendication 12, dans laquelle ladite région variable est une région variable de chaîne légère.

15. Utilisation conforme à la revendication 11, dans laquelle ladite molécule d'immunoglobuline comporte une chaîne lourde.

16. Utilisation conforme à la revendication 11, dans laquelle ladite molécule d'immunoglobuline comporte une chaîne légère.

17. Molécule d'acide nucléique comprenant un élément d'expression, spécifique pour cellules hématopoïétiques et fonctionnant dans un lymphocyte B, raccordé de manière opérationnelle à une séquence d'acide nucléique qui code un polypeptide hétérologue, lequel polypeptide hétérologue comporte deux épitopes T ou plus, lesquels épitopes T sont choisis parmi un épitope T CD4 et un épitope T CD8, deux épitopes T CD4 et deux épitopes T CD8.

18. Molécule d'acide nucléique conforme à la revendication 17, pour laquelle ledit polypeptide hétérologue comporte en outre un ou plusieurs épitope(s) B.

19. Molécule d'acide nucléique conforme à la revendication 17, dans laquelle ladite séquence d'acide nucléique code un polypeptide qui est exprimé sous la forme de produit de fusion avec une cytokine.

20. Molécule d'acide nucléique conforme à la revendication 19, ladite cytokine étant choisie dans l'ensemble formé par les suivantes : facteur GM-CSF (facteur de stimulation des colonies de granulocytes et macrophages), interleukine-2, interleukine-4, interféron gamma, interleukine-5, interleukine-6, interleukine-10 et interleukine-12.

21. Molécule d'acide nucléique conforme à l'une des revendications 17 à 20, dans laquelle ladite séquence d'acide nucléique code une molécule d'immunoglobuline qui comporte lesdits épitopes T au nombre de deux ou plus, lesquels épitopes T au nombre de deux ou plus sont insérés au sein d'une région CDR (région déterminant la complémentarité) de ladite molécule d'immunoglobuline.

22. Molécule d'acide nucléique conforme à la revendication 21, ladite immunoglobuline comprenant une région variable.

23. Molécule d'acide nucléique conforme à la revendication 22, ladite région variable étant une région variable de chaîne lourde.

24. Molécule d'acide nucléique conforme à la revendication 22, ladite région variable étant une région variable de chaîne légère.

25. Molécule d'acide nucléique conforme à la revendication 21, lesdits épitopes au nombre de deux ou plus étant insérés dans deux régions CDR.

26. Utilisation conforme à la revendication 1, pour laquelle la molécule d'acide nucléique est un vecteur non-viral et l'élément d'expression spécifique pour cellules hématopoïétiques est un élément d'expression spécifique pour lymphocytes B qui est opérationnellement raccordé à une séquence d'acide nucléique codant un polypeptide hétérologue, ladite molécule d'acide nucléique exprimant ledit polypeptide hétérologue quand elle a touché des lymphocytes B ciblés.

27. Utilisation conforme à la revendication 1, pour laquelle l'acide nucléique code deux épitopes T hétérologues ou plus.
